# EUROPEAN PATENT SPECIFICATION

(11) **EP 0 425 661 B1**
(45) Date of publication and mention of the grant of the patent: **11.10.2000**
(21) Application number: 90909224.9
(22) Date of filing: 16.05.1990
(51) Int. Cl.: C12N 15/00, C12P 19/34

(54) **A NEW METHOD FOR TAPPING THE IMMUNOLOGICAL REPERTOIRE**
NEUES VERFAHREN ZUR ERSCHLIESSUNG DES IMMUNOLOGISCHEN REPERTOIRS
NOUVEAU PROCEDE D'EXPLOITATION DU REPERTOIRE IMMUNOLOGIQUE

(30) Priority: 16.05.1989 US 353235; 16.05.1989 US 352927; 17.05.1989 US 353241; 17.05.1989 US 352884; 04.12.1989 US 446332; 20.03.1990 US 496522
(43) Date of publication of application: 08.05.1991
(60) Divisional application: 00103589.8
(73) Proprietor: STRATAGENE, La Jolla, CA 92037 (US); MEDICAL RESEARCH COUNCIL, London W1N 4AL (GB); THE SCRIPPS RESEARCH INSTITUTE, La Jolla, CA 92037 (US)
(72) Inventor: LERNER, Richard, A., La Jolla, CA 92037 (US); SORGE, Joseph, A., Rancho Santa Fe, CA 92067 (US)
(74) Representative: Brasnett, Adrian Hugh
(86) International application number: US9002836
(87) International publication number: WO9014430

(56) References cited:
- WO-A-88/06630
- WO-A-88/09344
- WO-A-89/01526
- US-A- 4 642 334
- US-A- 4 656 134
- US-A- 4 800 159
- PROCEEDINGS OF THE NATIONAL ACADEMY OF SCIENCES OF USA. vol. 10, 15 May 1989, WASHINGTON US pages 3833 - 3837; Orlandi, R. et al.: 'Cloning Immunoglobulin variable domains for expression by the polymerase chain reaction.'
- BIOCHEMICAL AND BIOPHYSICAL RESEARCH COMMUNICATIONS. vol. 3, 15 May 1989, DULUTH, MINNESOTA US pages 1250 - 1256; Larrick, J.W. et al.: 'Rapid cloning of rearranged Immunoglobulin genes from human hybridoma cells using mixed primers and the polymerase chain reaction.'
- SCIENCE. vol. 246, 8 December 1989, LANCASTER, PA US pages 1275 - 1281; Huse, W.D. et al.: 'Generation of a large combinatorial library of the Immunoglobulin repertoire in Phage lambda.'
- PROCEEDINGS OF THE NATIONAL ACADEMY OF SCIENCES OF USA. vol. 86, August 1989, WASHINGTON US pages 5728 - 5732; Sastry, L. et al.: 'Cloning of the immunological repertoire in Esherichia coli for generation of monoclonal catalytic antibodies : construction of a heavy chain variable region-specific cDNA library'
- Nucleic Acids Research, Vol. 16, No. 15, issued 1988, Short, "ZAP; a Bacteriophaged Expression Vector with in Vivo Excision Properties" pages 7583-7600. see fig. 1A.
- Science, Vol. 241, issued 02 September 1988, JANDA et al, "Induction of an Antibdy that Catalyzes the Hydrolysis of an Amide Bond", pages 1188-1191. see page 1188, paragraphs 1 & 2, see Abstract.
- Science, Vol. 234, issued 19 December 1986, PUELACH et al, "Selective Chemical Ctalysis by an Antibody", pages 1570-1573. see page 1570 and 1572 underlined sections.
- Science, Vol. 234, issued 19 December 1986, TRAMONTINO et al, "Catalytic Antibodes" pages 1566-1570, see page 1569, columns 2 & 3.

## Description

### Technical Field

The present invention relates to a method for isolating a gene coding for an antibody heavy chain variable region (VH) or antibody light chain variable region (VL) having catalytic activity.

### Background

Binding phenomena between ligands and receptors play many crucial roles in biological systems. Exemplary of such phenomena is the the binding of a substrate to an enzyme that acts upon it such as between a protein and a protease like trypsin.

Many drugs and other therapeutic agents are also believed to be dependent upon binding phenomena.

Ligands such as man-made drugs, like morphine and its derivatives, and those that are naturally present in biological systems such as endorphins and hormones bind to receptors that are naturally present in biological systems, and will be treated together herein. Such binding can lead to a number of the phenomena of biology, including particularly the hydrolysis of amide and ester bonds as where proteins are hydrolyzed into constituent polypeptides by an enzyme such as trypsin or papain or where a fat is cleaved into glycerine and three carboxylic acids, respectively. In addition, such binding can lead to formation of amide and ester bonds in the formation of proteins and fats, as well as to the formation of carbon to carbon bonds and carbon to nitrogen bonds.

An exemplary receptor-producing system in vertebrates is the immune system. The immune system of a mammal is one of the most versatile biological systems as probably greater than 1.0 x 10⁷ receptor specificities, in the form of antibodies, can be produced. Indeed, much of contemporary biological and medical research is directed toward tapping this repertoire. During the last decade there has been a dramatic increase in the ability to harness the output of the vast immunological repertoire. The development of the hybridoma methodology by Kohler and Milstein has made it possible to produce monoclonal antibodies, i.e., a composition of antibody molecules of a single specificity, from the repertoire of antibodies induced during an immune response.

Unfortunately, current methods for generating monoclonal antibodies are not capable of efficiently surveying the entire antibody response induced by a particular immunogen. In an individual animal there are at least 5-10,000 different B-cell clones capable of generating unique antibodies to a small relatively rigid immunogens, such as, for example dinitrophenol. Further, because of the process of somatic mutation during the generation of antibody diversity, essentially an unlimited number of unique antibody molecules may be generated. In contrast to this vast potential for different antibodies, current hybridoma methodologies typically yield only a few hundred different monoclonal antibodies per fusion.

Other difficulties in producing monoclonal antibodies with the hybridoma methodology include genetic instability and low production capacity of hybridoma cultures. One means by which the art has attempted to overcome these latter two problems has been to clone the immunoglobulin-producing genes from a particular hybridoma of interest into a prokaryotic expression system. See, for example, Robinson et al., PCT Publication No. WO 89/0099; Winter et al., European Patent Publication No. 0239400; Reading, U.S. Patent No. 4,714,681; and Cabilly et al., European Patent Publication No. 0125023.

The immunologic repertoire of vertebrates has recently been found to contain genes coding for immunoglobulins having catalytic activity. Tramontano et al., Sci., 234:1566-1570 (1986); Pollack et al., Sci., 234:1570-1573 (1986); Janda et al., Sci., 241:1188-1191 (1988); and Janda et al., Sci., 244:437-440 (1989). The presence of, or the ability to induce the repertoire to produce, antibodies molecules capable of a catalyzing chemical reaction, i.e., acting like enzymes, had previously been postulated almost 20 years ago by W. P. Jencks in Catalysis in Chemistry and Enzymology, McGraw-Hill, N.Y. (1969).

It is believed that one reason the art failed to isolate catalytic antibodies from the immunological repertoire earlier, and its failure to isolate many to date even after their actual discovery, is the inability to screen a large portion of the repertoire for the desired activity. Another reason is believed to be the bias of currently available screening techniques, such as the hybridoma technique, towards the production of high affinity antibodies inherently designed for participation in the process of neutralization, as opposed to catalysis.

### Brief Summary of the Invention.

The present invention provides a novel method of method of producing a V_{H} or V_{L} catalytic-receptor-coding nucleic acid, which method comprises:
(a) synthesizing a conserved V_{H} or V_{L} receptor-coding gene library containing at least 10³ difference V_{H} or V_{L} receptor-coding DNA homologs by:
   (i) separating the strands of a repertoire of conserved V_{H} or V_{L} receptor-coding genes, said repertoire comprising double-stranded nucleic acids each containing a V_{H} or V_{L} receptor-coding strand annealed to a complementary strand;
   (ii) treating said separated strands, under conditions suitable for polymerase chain reaction amplification, with first and second polynucleotide synthesis primers, each of said first primers having a nucleotide sequence capable of hybridizing to a sequence conserved among said V_{H} or V_{L} receptor-coding strands, and each of said second primers having a nucleotide sequence capable of hybridizing to a sequence conserved among said complementary strands, said primers being capable of priming the amplification of a plurality of different V_{H} or V_{L} receptor-coding DNA homologs from said V_{H} or V_{L} receptor-coding gene repertoire, said treating producing said conserved receptor-coding gene library; and
(b) segregating from said library a V_{H} or V_{L} receptor-coding nucleic acid coding for a catalytic receptor.

In one embodiment, said segregating comprises:
(a) operatively linking for expression each of a plurality of said different V_{H}-coding DNA homologs to an expression vector, thereby forming a plurality of different V_{H}-expression vectors;
(b) transforming a population of host cells compatible with said expression vector with a plurality of said different V_{H}-expression vectors to produce a transformed population of host cells whose members contain said V_{H}-expression vectors;
(c) culturing said transformed population under conditions for expressing the receptors coded for by said V_{H}-coding DNA homologs;
(d) assaying the members of said transformed population for expression of a receptor capable of binding a preselected ligand, thereby identifying transformants containing said V_{H}-coding DNA homolog; and
(e) segregating an identified transformant of step (d) from said population, thereby producing said conserved V_{H}-coding nucleic acid.

The invention also provides a method of producing a catalytic receptor comprising:
(a) operatively linking for expression a gene isolated according to the above methods of the invention to a suitable expression vector to form V_{H}-expression vector;
(b) transforming a host cell compatible with said expression vector to produce a transformant;
(c) culturing said transformant under conditions for expressing the catalytic receptor coded for by said V_{H}-coding DNA homolog, thereby producing said catalytic receptor in said culture; and
(d) recovering from said culture said catalytic receptor.

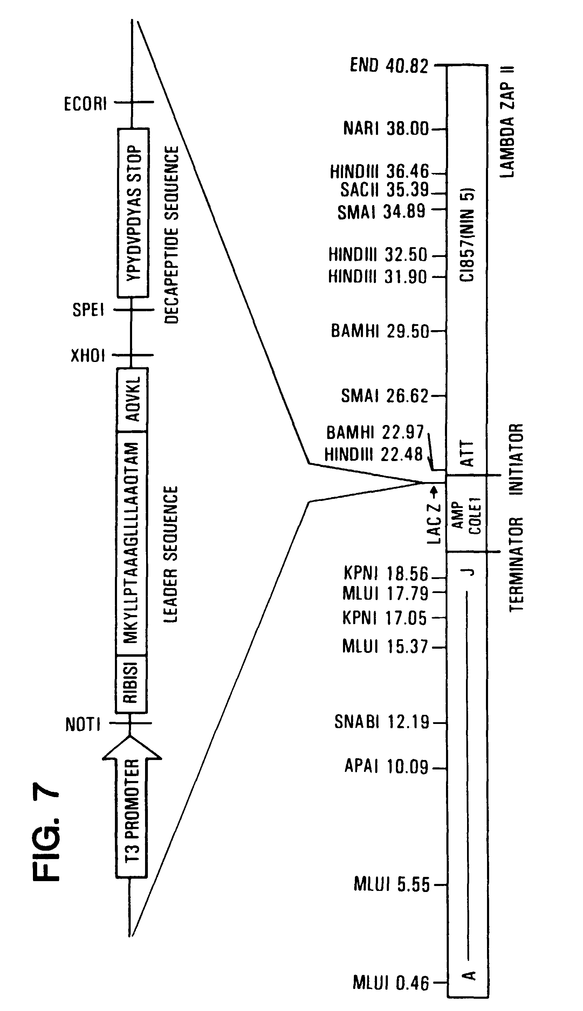

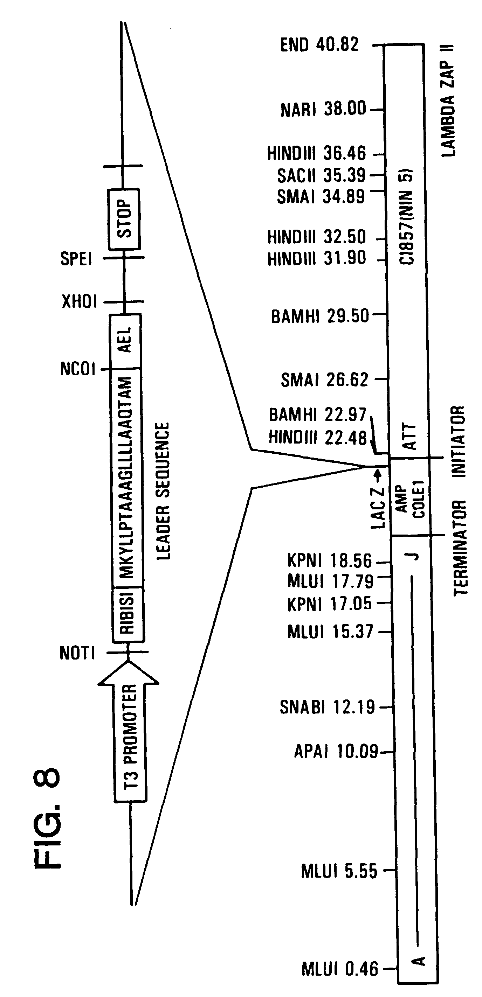

The invention also provides the bacterial expression vector Lambda Zap II V_{H}, as depicted in Figure 7 and the bacterial expression vector Lambda Zap II V_{L}, as depicted in Figure 8. complementary strands, said primers being capable of priming the amplification of a plurality of different first polypeptide-coding DNA homologs from said first polypeptide-coding gene repertoire, said treating producing said first polypeptide-coding gene library;
(b) synthesizing a second polypeptide-coding gene library containing a plurality of different second polypeptide-coding DNA homologs by:
   (i) separating the strands of a repertoire of second polypeptide-coding genes, said repertoire comprising double-stranded nucleic acids each containing a second polypeptide-coding strand annealed to a second complementary strand;
   (ii) treating said separated strands, under conditions suitable for polymerase chain reaction amplification, with third and fourth polynucleotide synthesis primers, each of said third primers having a nucleotide sequence capable of hybridizing to a sequence conserved among said second polypeptide-coding strands, and each of said fourth primers having a nucleotide sequence corresponding to a sequence conserved among said second complementary strands, said primers being capable of priming the amplification of a plurality of different second polypeptide-coding DNA homologs from said second polypeptide-coding gene repertoire, said treating producing said second polypeptide-coding gene library;
(c) forming a diverse library of coexpression vectors by treating expression vector molecules adapted for ligation to the first polypeptide- and second polypeptide-coding DNA homologs of steps (a) (ii) and (b) (ii), respectively, with a diverse plurality of said first polypeptide-coding DNA homologs and a diverse plurality of said second polypeptide-coding DNA homologs, under conditions suitable for DNA ligation to produce a plurality of different coexpression vectors, each of said different coexpression vectors being capable of expressing a heterodimeric receptor molecule comprising a combination of first and second polypeptides that is different from the combination of first and second polypeptides forming heterodimeric receptor molecules expressed by any other of said different coexpression vectors; and
(d) segregating from said diverse library of coexpression vectors a coexpression vector capable of expressing an antibody of predetermined specificity.

The invention further provides a double stranded DNA vector containing a V_{H}-DNA coding sequence and a second DNA sequence comprising a V_{L}-coding DNA sequence, said vector providing expression of said DNA sequences in a host cell.

### Brief Description of the Drawings

In the drawings forming a portion of this disclosure;

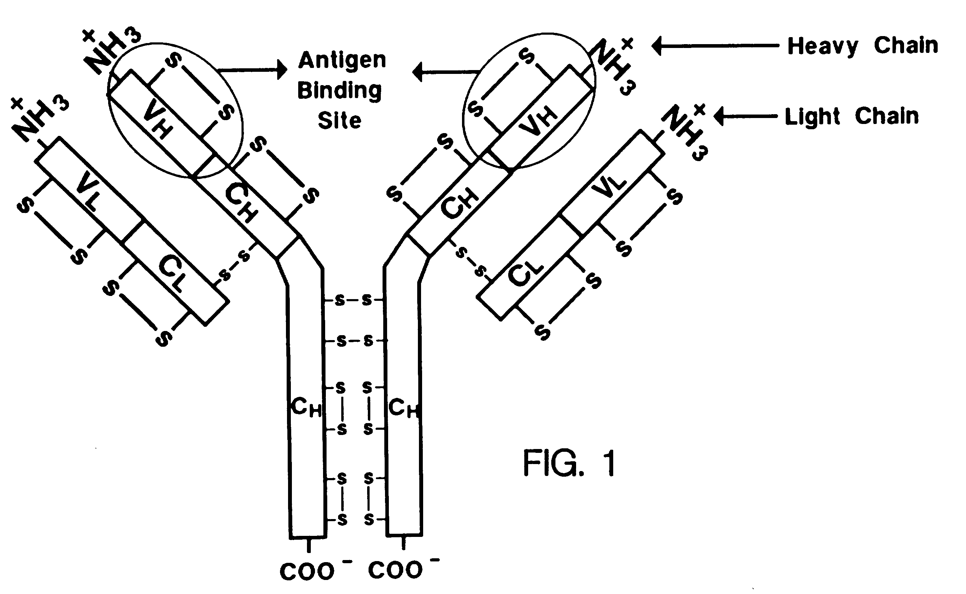

Figure 1 Illustrates a schematic diagram of the immunoglobulin molecule showing the principal structural features. The circled area on the heavy chain represents the variable region (V_{H}), a polypeptide containing a biologically active (ligand binding) portion of that region, and a gene coding for that polypeptide, are produced by the methods of the present invention. Sequences L03, L35, L47 and L48 could not be classified into any predefined subgroups.

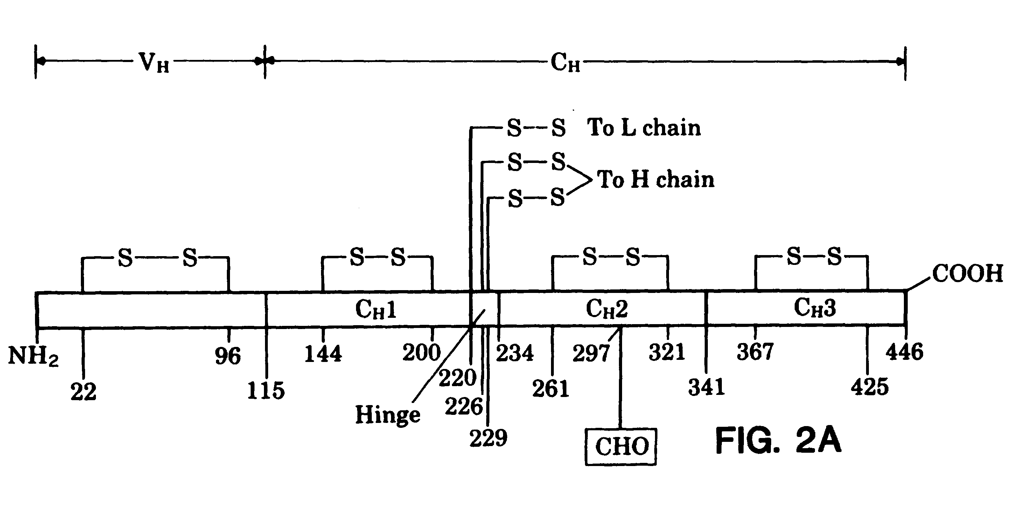

Figure 2A Diagrammatic sketch of an H chain of human IgG (IgG1 subclass). Numbering is from the N-terminus on the left to the C-terminus on the right. Note the presence of four domains, each containing an intrachain disulfide bond (S-S) spanning approximately 60 amino acid residues. The symbol CHO stands for carbohydrate. The V region of the heavy (H) chain (V_{H}) resembles V_{L} in having three hypervariable CDR (not shown).

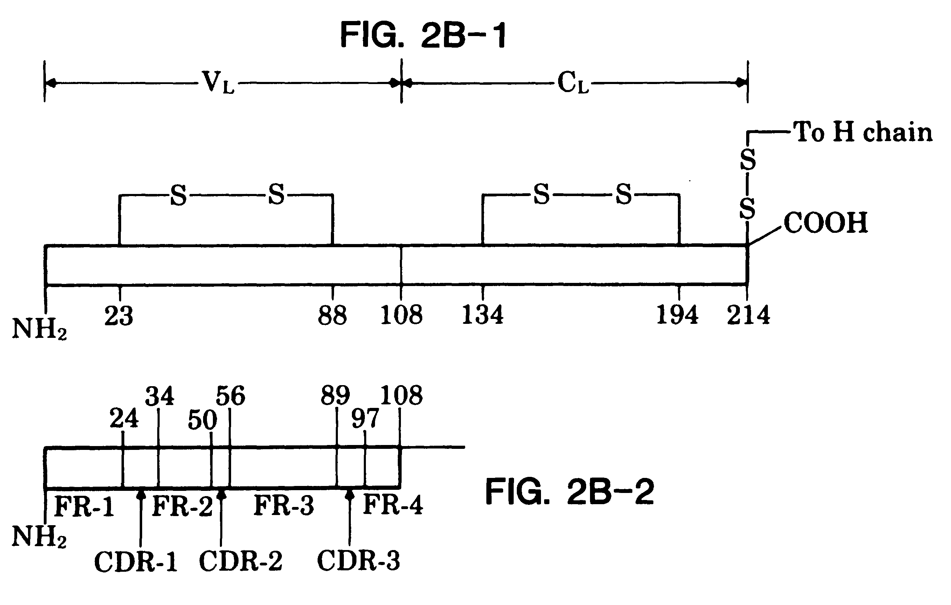

Figure 2B Diagrammatic sketch of a human K chain (Panel 1). Numbering is from the N-terminus on the left to the C-terminus on the right. Note the intrachain disulfide bond (S-S) spanning about the same number of amino acid residues in the V_{L} and C_{L} domains. Panel 2 shows the locations of the complementarity-determining regions (CDR) in the V_{L} domain. Segments outside the CDR are the framework segments (FR).

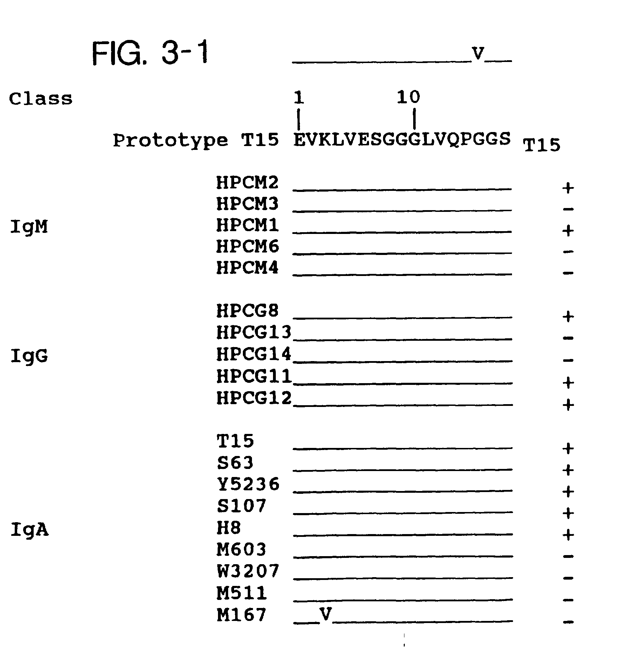

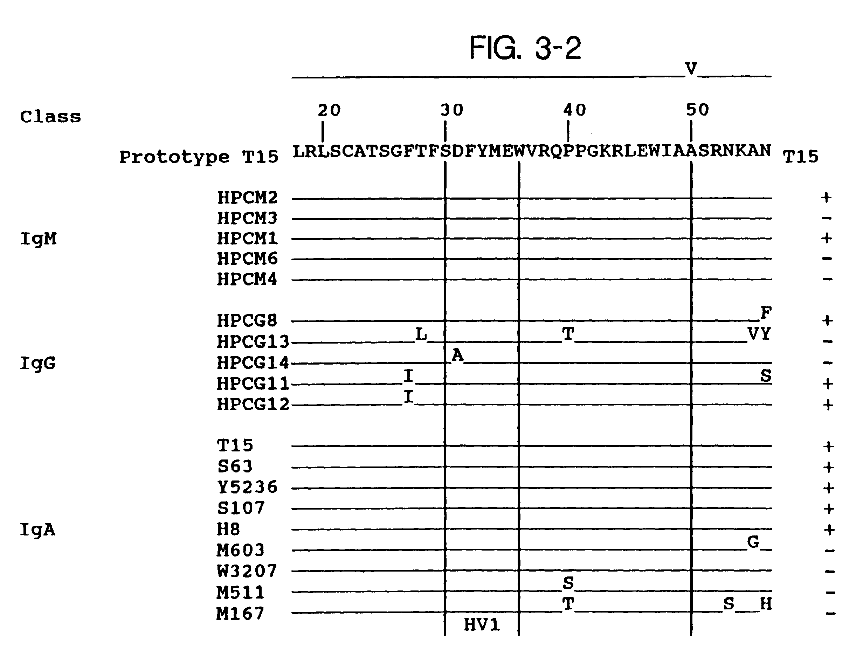

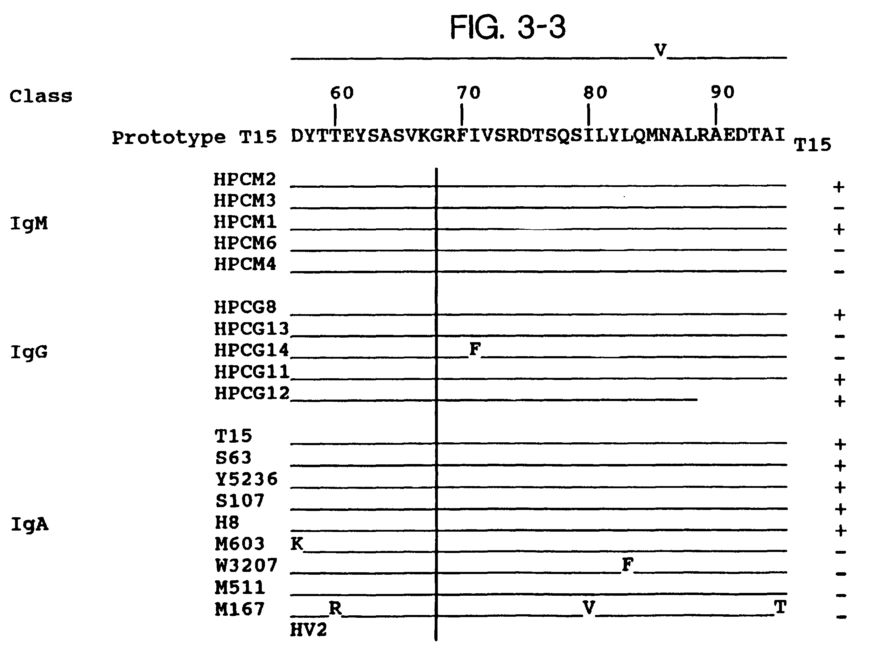

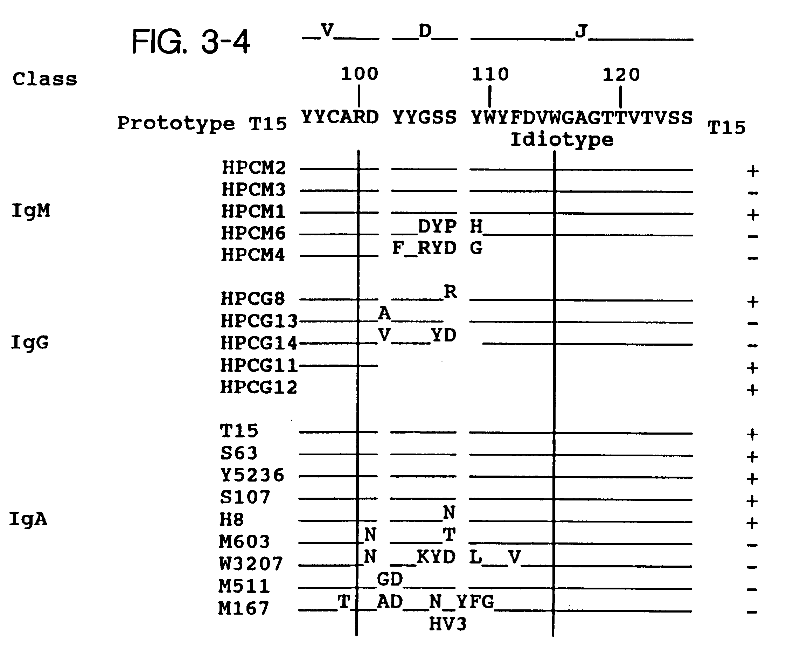

Figure 3 Amino acid sequence of the V_{H} regions of 19 mouse monoclonal antibodies with specificity for phosphorylcholine. The designation HP indicates that the protein is the product of a hybridoma. The remainder are myeloma proteins. (From Gearhart et al., Nature, 291:29, 1981.)

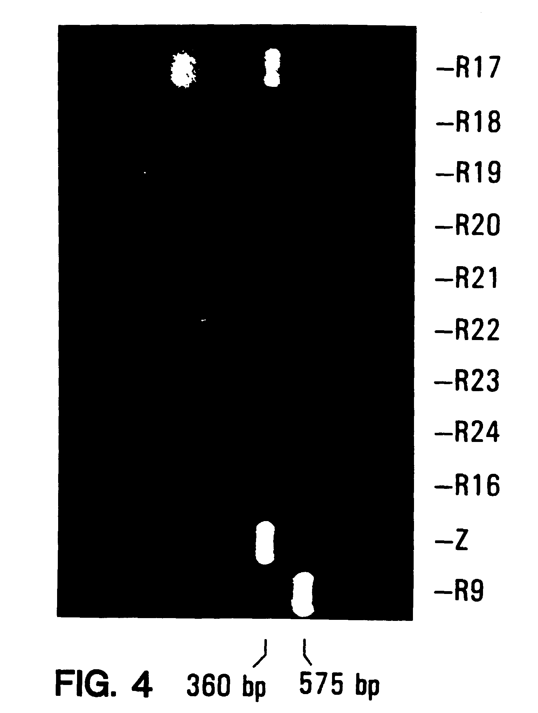

Figure 4 Illustrates the results obtained from PCR amplification of mRNA obtained from the spleen of a mouse immunized with FITC. Lanes R17-R24 correspond to amplification reactions with the unique 5' primers (2-9, Table 1) and the 3' primer (12, Table 1), R16 represents the PCR reaction with the 5' primer containing inosine (10, Table 1) and 3' primer (12, Table 1). Z and R9 are the amplification controls; control Z involves the amplification of V_{H} from a plasmid (PLR2) and R9 represents the amplification from the constant regions of spleen mRNA using primers 11 and 13 (Table 1).

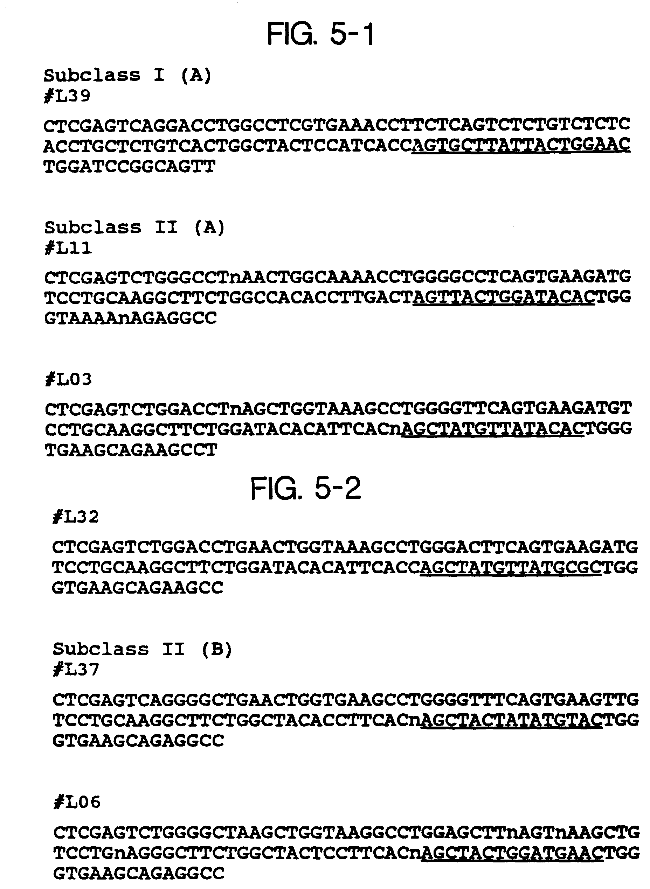

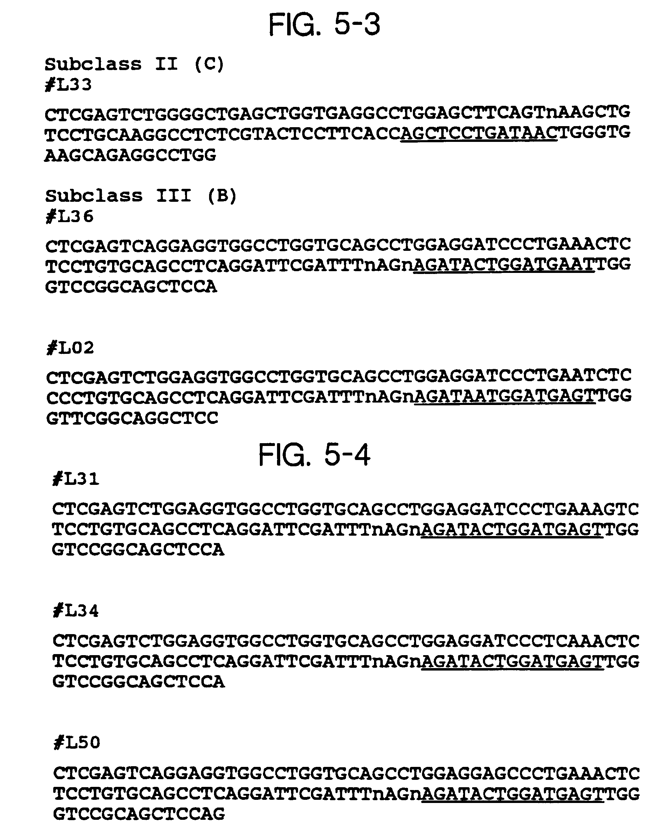

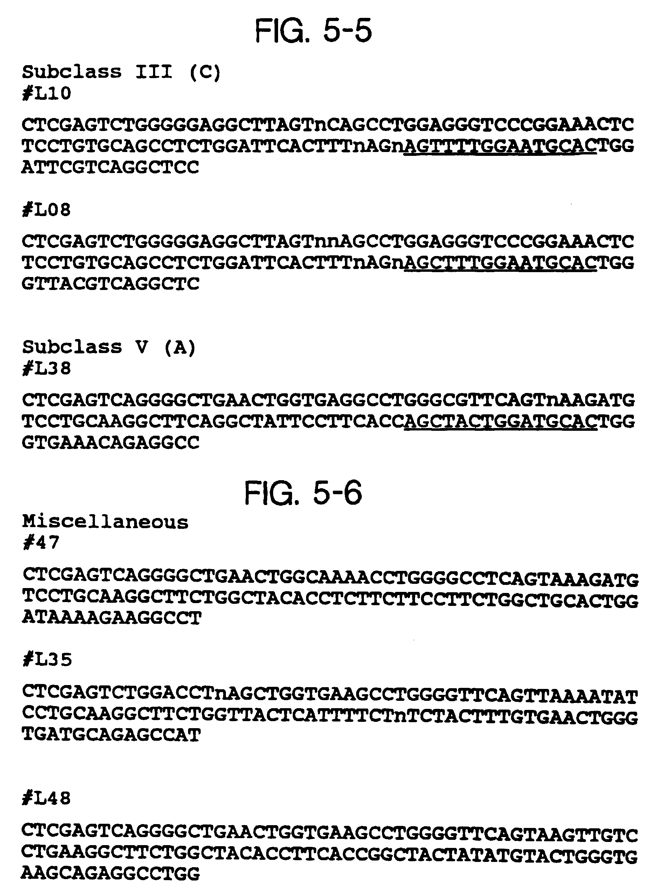

Figure 5 Nucleotide sequences are clones from the cDNA library of the PCR amplified V_{H} regions in Lambda ZAP. The N-terminal 110 bases are listed here and the underlined nucleotides represent CDR1 (complementary determining region).

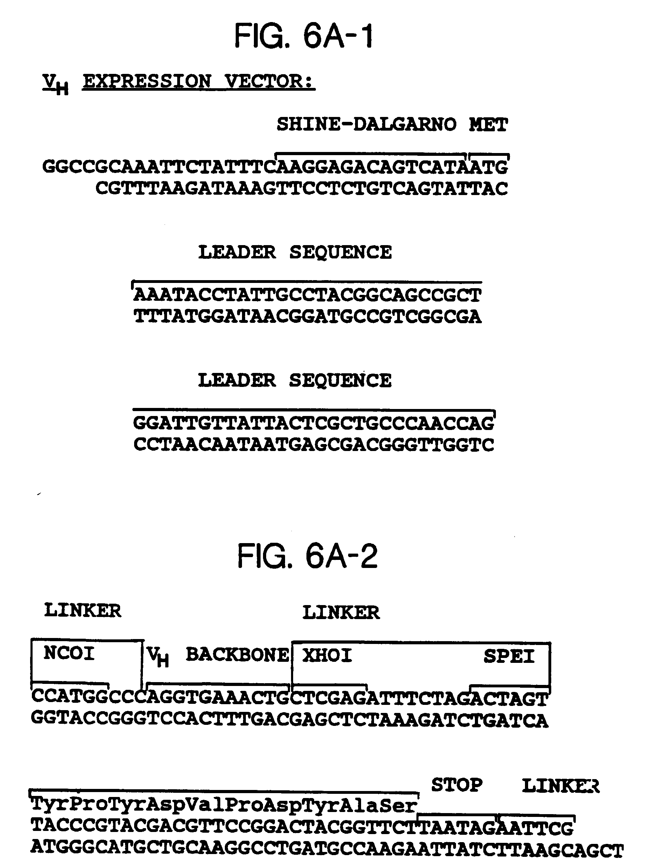

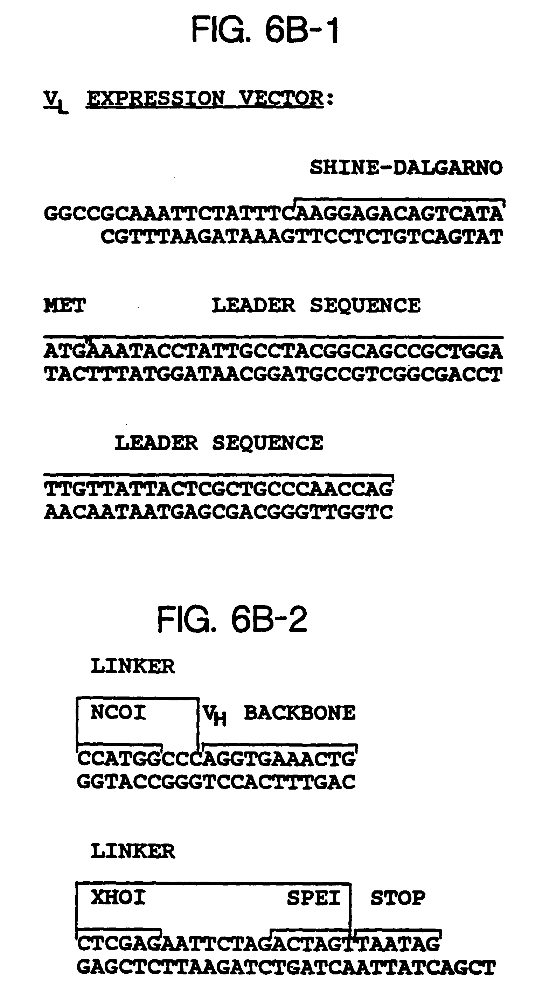

Figure 6 The sequence of the synthetic DNA insert inserted into Lambda ZAP to produce Lambda Zap II V_{H} (Panel A) and Lambda Zap V_{L} (Panel B) expression vectors. The various features required for this vector to express the V_{H} and V_{L}-coding DNA homologs include the Shine-Dalgarno ribosome binding site, a leader sequence to direct the expressed protein to the periplasm as described by Mouva et al., J. Biol. Chem., 255:27, 1980, and various restriction enzyme sites used to operatively link the V_{H} and V_{L} homologs to the expression vector. The V_{H} expression-vector sequence also contains a short nucleic acid sequence that codes for amino acids typically found in variable regions heavy chain (V_{H} Backbone). This V_{H} Backbone is just upstream and in the proper reading as the V_{H} DNA homologs that are operatively linked into the Xho I and Spe I. The V_{L} DNA homologs are operatively linked into the V_{L} sequence (Panel B) at the Nco I and Spe I restriction enzyme sites and thus the V_{H} Backbone region is deleted when the V_{L} DNA homologs are operatively linked into the V_{L} vector.
Figure 7 The major features of the bacterial expression vector Lambda Zap II V_{H} (V_{H}-expression vector) are shown. The synthetic DNA sequence from Figure 6 is shown at the top along with the T₃ polymerase promoter from Lambda Zap II. The orientation of the insert in Lambda Zap II is shown. The V_{H} DNA homologs are inserted into the Xho I and Spe I restriction enzyme sites. The V_{H} DNA are inserted into the Xho I and Spe I site and the read through transcription produces the decapeptide epitope (tag) that is located just 3' of the cloning sites.
Figure 8 The major features of the bacterial expression vector Lambda Zap II V_{L} (V_{L} expression vector) are shown. The synthetic sequence shown in Figure 6 is shown at the top along with the T₃ polymerase promoter from Lambda Zap II. The orientation of the insert in Lambda Zap II is shown. The V_{L} DNA homologs are inserted into the phagemid that is produced by the in vivo excision protocol described by Short et al., Nucleic Acids Res., 16:7583-7600, 1988. The V_{L} DNA homologs are inserted into the Nco I and Spe I cloning sites of the phagemid.

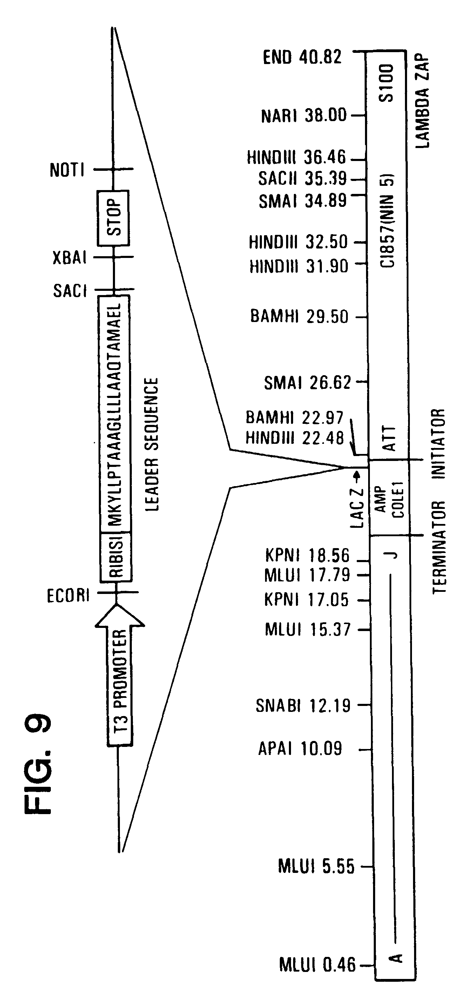

Figure 9 A modified bacterial expression vector Lambda Zap II V_{L}II. This vector is constructed by inserting this synthetic DNA sequence, 
TGAATTCTAAACTAGTCGCCAAGGAGACAGTCATAATGAA
TCGAACTTAAGATTTGATCAGCGGTTCCTCTGTCAGTATTACTT

ATACCTATTGCCTACGGCAGCCGCTGGATTGTTATTACTCGCTG
TATGGATAACGGATGCCGTCGGCGACCTAACAATAATGAGCGAC

CCCAACCAGCCATGGCCGAGCTCGTCAGTTCTAGAGTTAAGCGGCCG
GGGTTGGTCGGTACCGGCTCGAGCAGTCAAGATCTCAATTCGCCGGCAGCT
 into Lambda Zap II that has been digested with the restriction enzymes Sac I and Xho I. This sequence contains the Shine-Dalgarno sequence (Ribosome binding site), the leader sequence to direct the expressed protein to the periplasm and the appropriate nucleic acid sequence to allow the V_{L} DNA homologs to be operatively linked into the SacI and Xba I restriction enzyme sites provided by this vector.

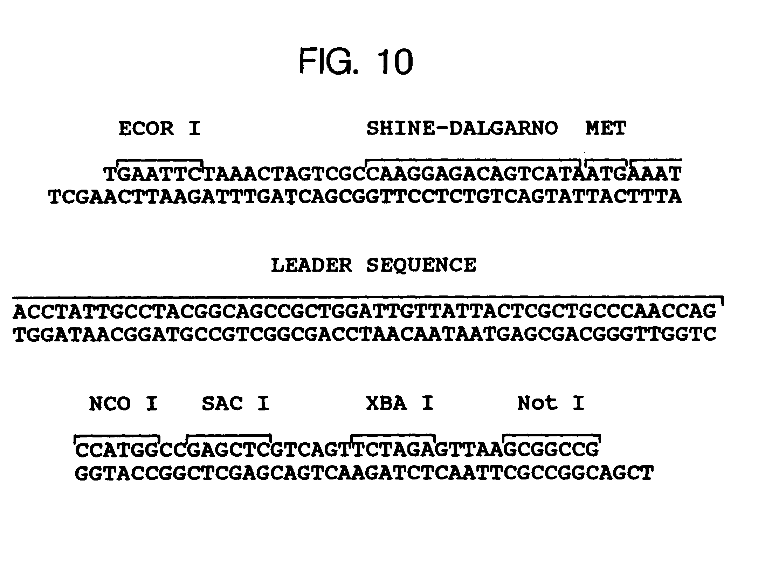

Figure 10 The sequence of the synthetic DNA segment inserted into Lambda Zap II to produce the lambda V_{L}II-expression vector. The various features and restriction endonuclease recognition sites are shown.

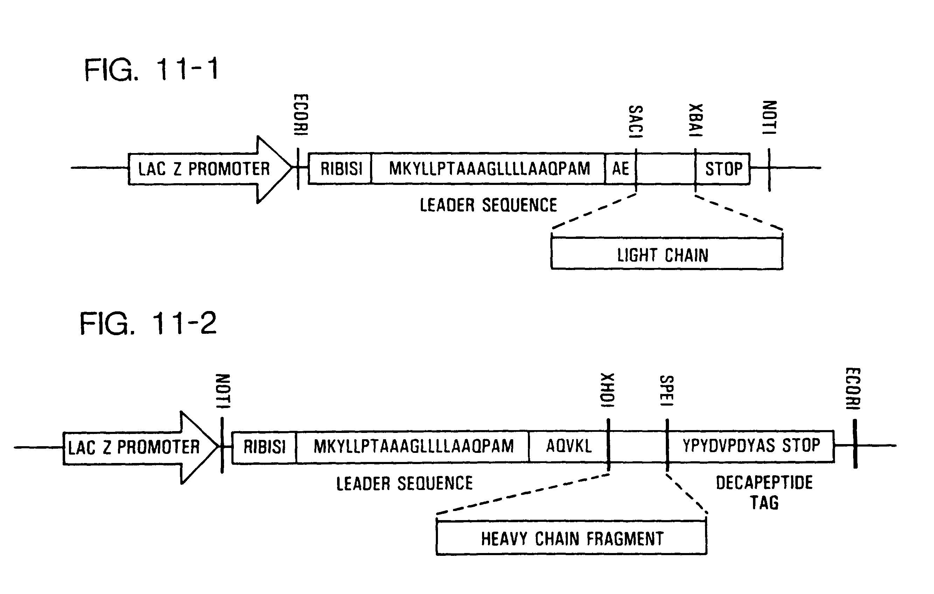

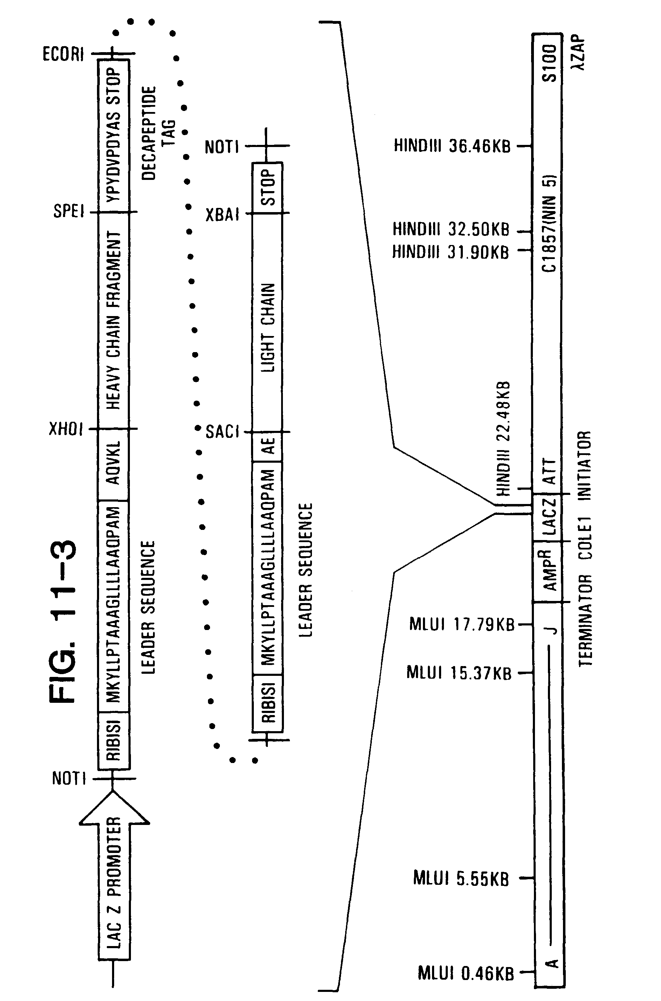

Figure 11 The vectors for expressing V_{H} and V_{L} separately and in combination are shown. The various essential components of these vectors are shown. The light chain vector or V_{L} expression vector can be combined with the V_{H} expression vector to produce a combinatorial vector containing both V_{H} and V_{L} operatively linked for expression to the same promoter.

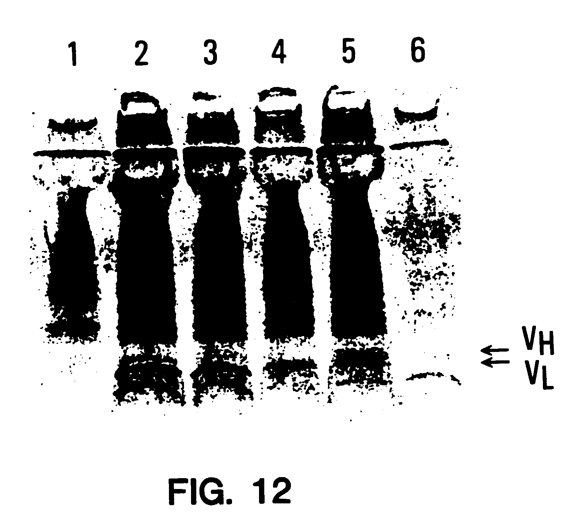

Figure 12 The labelled proteins immunoprecipitated from E. coli containing a V_{H} and a V_{L} DNA homolog are shown. In lane 1, the background proteins immunoprecipitated from E. coli that do not contain a V_{H} or V_{L} DNA homolog are shown. Lane 2 contains the V_{H} protein immunoprecipitated from E. coli containing only a V_{H} DNA homolog. In lanes 3 and 4, the co-migration of a V_{H} protein a V_{L} protein immunoprecipitated from E. coli containing both a V_{H} and a V_{L} DNA homolog is shown. In lane 5 the presence of V_{H} protein and V_{L} protein expressed from the V_{H} and V_{L} DNA homologs is demonstrated by the two distinguishable protein species. Lane 5 contains the background proteins immunoprecipitated by anti-E. coli antibodies present in mouse ascites fluid.

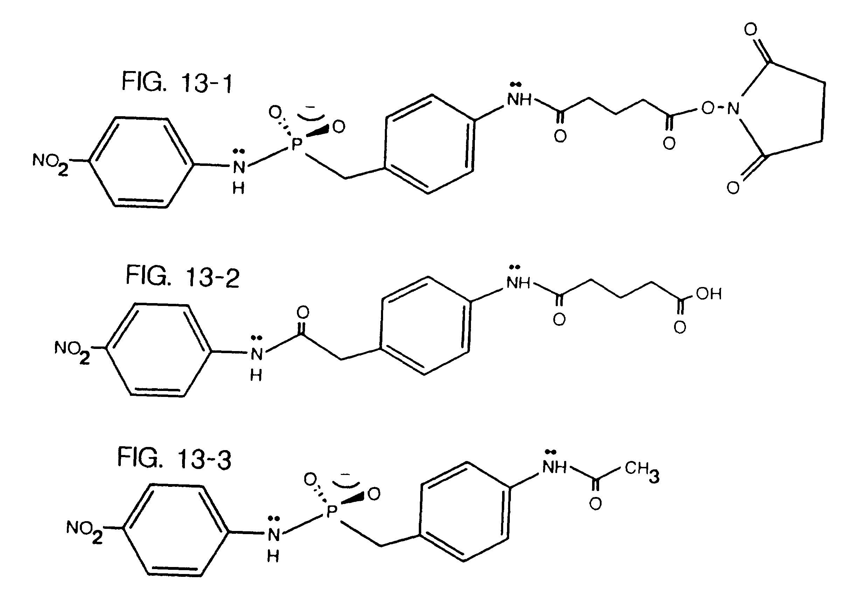

Figure 13 The transition state analogue (formula 1) which induces antibodies for hydrolyzing carboxamide substrate (formula 2). The compound of formula 1 containing a glutaryl spacer and a N-hydroxysuccinimide-linker appendage is the form used to couple the hapten (formula 1) to protein carriers KLH and BSA, while the compound of formula 3 is the inhibitor. The phosphonamidate functionality is a mimic of the stereo-electronic features of the transition state for hydrolysis of the amide bond.

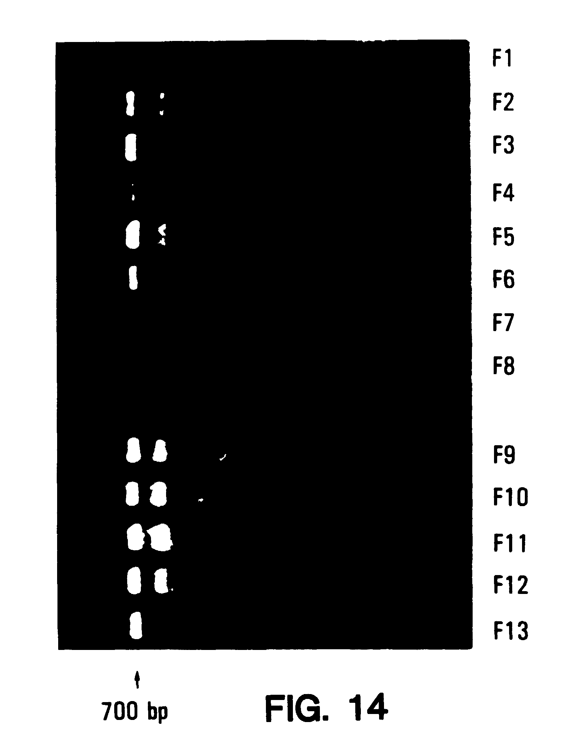

Figure 14 PCR amplification of Fd and kappa regions from the spleen mRNA of a mouse immunized with NPN is illustrated. Amplification was performed as described in Example 18 using RNA cDNA hybrids obtained by the reverse transcription of the mRNA with primer specific for amplification of light chain sequences (Table 2) or heavy chain sequences (Table 1). Lanes F1-F8 represent the product of heavy chain amplification reactions with one of each of the eight 5' primers (primers 2-9, Table 1) and the unique 3' primer (primer 15, Table 2). Light chain (k) amplifications with the 5' primers (primers 3-6, and 12, respectively, Table 2) and the appropriate 3' primer (primer 13, Table 2) are shown in lanes F9-F13. A band of 700 bps is seen in all lanes indicating the successful amplification of Fd and k regions.

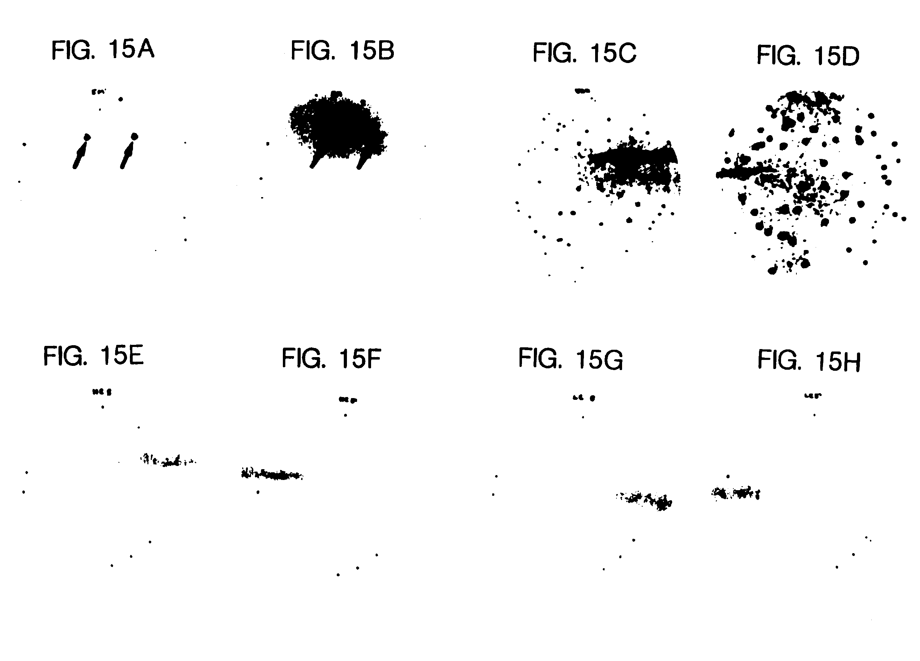

Figure 15 The screening of phage libraries for antigen binding is depicted according to Example 18C. Duplicate plaque lifts of Fab (filters A,B), heavy chain (filters E,F) and light chain (filters G,H) expression libraries were screened against ¹²⁵I-labelled BSA conjugated with NPN at a density of approximately 30,000 plaques per plate. Filters C and D illustrate the duplicate secondary screening of a cored positive from a primary filter A (arrows) as discussed in the text.
   Screening employed standard plaque lift methods. XL1 Blue cells infected with phage were incubated on 150mm plates for 4h at 37°, protein expression induced by overlay with nitrocellulose filters soaked in 10mM isopropyl thiogalactoside (IPTG) and the plates incubated at 25° for 8h. Duplicate filters were obtained during a second incubation employing the same conditions. Filters were then blocked in a solution of 1% BSA in PBS for 1h before incubation with rocking at 25° for 1h with a solution of ¹²⁵I-labelled BSA conjugated to NPN (2 x 10⁶ cpm ml⁻¹; BSA concentration at 0^{·}1 M; approximately 15 NPN per BSA molecule) in 1% BSA/PBS. Background was reduced by pre-centrifugation of stock radiolabelled BSA solution at 100,000 g for 15 min and pre-incubation of solutions with plaque lifts from plates containing bacteria infected with a phage having no insert. After labeling, filters were washed repeatedly with PBS/0.05% Tween 20 before development of autoradiographs overnight.

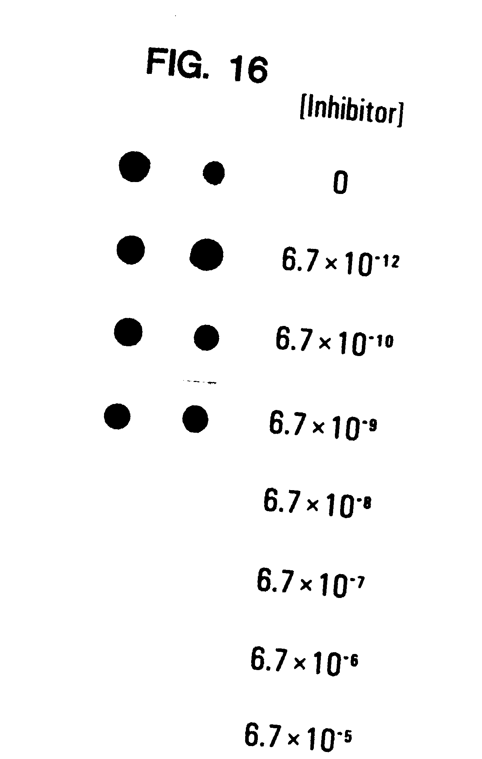

Figure 16 The specificity of antigen binding as shown by competitive inhibition is illustrated according to Example 18C. Filter lifts from positive plaques were exposed to ¹²⁵I-BSA-NPN in the presence of increasing concentrations of the inhibitor NPN.
   In this study a number of phages correlated with NPN binding as in Figure 15 were spotted (about 100 particles per spot) directly onto a bacterial lawns. The plate was then overlaid with an IPTG-soaked filter and incubated for 19h at 25°. The filter were then blocked in 1% BSA in PBS prior to incubation in ¹²⁵I-BSA-NPN as described previously in Figure 15 except with the inclusion of varying amounts of NPN in the labeling solution. Other conditions and procedures were as in Figure 15. The results for a phage of moderate affinity are shown in duplicate in the figure. Similar results were obtained for four other phages with some differences in the effective inhibitor concentration ranges.

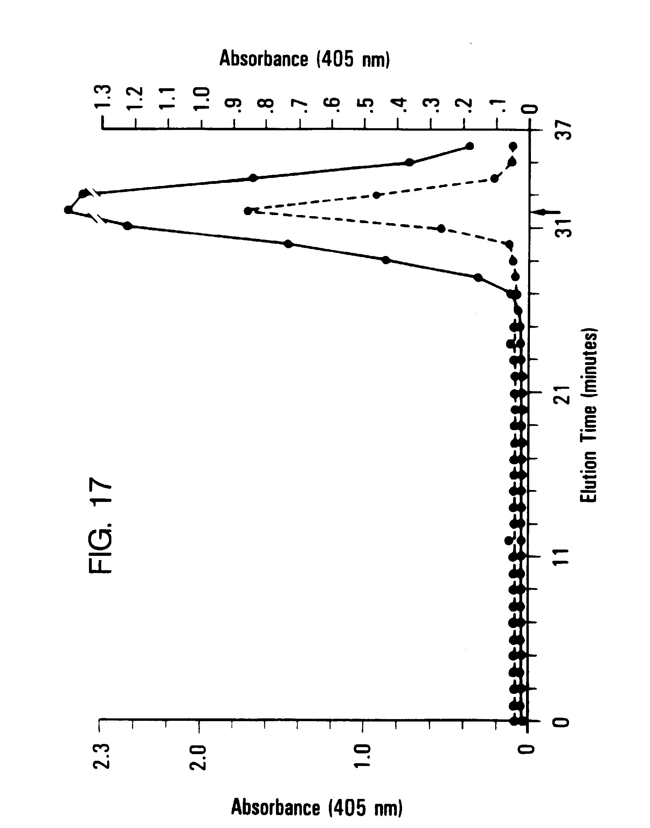

Figure 17 The characterization of an antigen binding protein is illustrated according to Example 18D. The concentrated partially purified bacterial supernate of an NPN-binding clone was separated by gel filtration and aliquots from each fraction applied to microtitre plates coated with BSA-NPN. Addition of either anti-decapeptide (---) or anti-kappa chain (―) antibodies conjugated with alkaline phosphatase was followed by color development. The arrow indicates the position of elution of a known Fab fragment. The results show that antigen binding is a property of 50 kD protein containing both heavy and light chains.
   Single plaques of two NPN-positive clones (Figure 15) were picked and the plasmid containing the heavy and light chain inserts excised (19). 500 ml cultures in L-broth were inoculated with 3 ml of a saturated culture containing the excised plasmids and incubated for 4h at 37 . Proteins synthesis was induced by the addition of IPTG to a final concentration of 1mM and the cultures incubated for 10h at 25°. 200 ml of cells supernate were concentrated to 2 ml and applied to a TSK-G4000 column. 50 µl aliquots from the eluted fractions were assayed by ELISA.
   For ELISA analysis, microtitre plates were coated with BSA-NPN at 1 ug/ml, 50 µl samples mixed with 50 µl PBS-Tween 20 (0.05%)-BSA (0.1%) added and the plates incubated for 2h at 25°. After washing with PBS-Tween 20-BSA, 50 µl of appropriate concentrations of a rabbit anti-decapeptide antibody (20) and a goat anti-mouse kappa light chain (Southern Biotech) antibody conjugated with alkaline phosphatase were added and incubated for 2h at 25°. After further washing, 50 µl of p-nitrophenyl phosphate (1 mg/ml in 0.1M tris pH 9.5 containing 50 mM MgC12) were added and the plates incubated for 15-30 min before reading the OD at 405 nm.

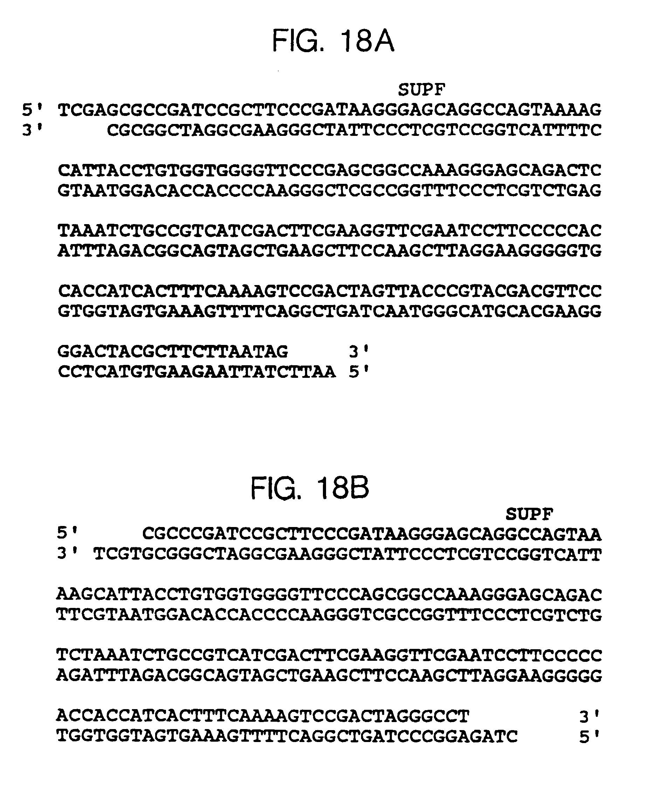

Figure 18 The sequence of the synthetic DNA insert inserted into Lambda Zap II V_{H} to produce the selectable V_{H} expression vector (panel A) and Lambda Zap II V_{L} II according to Example 17 to produce the selectable V_{L} expression vector (panel B).
Figure 19
   (A) The major features of the selectable V_{L} expression vector are shown in panel A. The feature of the synthetic DNA sequence from Figure 18A is shown at the top along with the T₃ polymerase promoter from Lambda Zap II. The orientation of the insert in Lambda Zap II is shown. The V_{H} DNA homologs are inserted into the Xho I and Spe I restriction enzyme sites. The V_{H} DNA homologs are inserted into the Xho I and Spe I site and the read through transcription produces the decapeptide epitope (tag) that is located just 3' of the cloning sites.
(B) The major features of the bacterial expression vector Lambda Zap II V_{H} (V_{H}-expression vector) are shown. the synthetic DNA sequence from Figure 6 is shown at the top along with the T₃ polymerase promoter from Lambda Zap II. The orientation of the insert in Lambda Zap II is shown. The V_{H} DNA homologs are inserted into the Xho I and Spe I restriction enzyme sites. The V_{H} DNA are inserted into the Xho I and Spe I site and the read through transcription produces the decapeptide epitope (tag) that is located just 3' of the cloning sites.

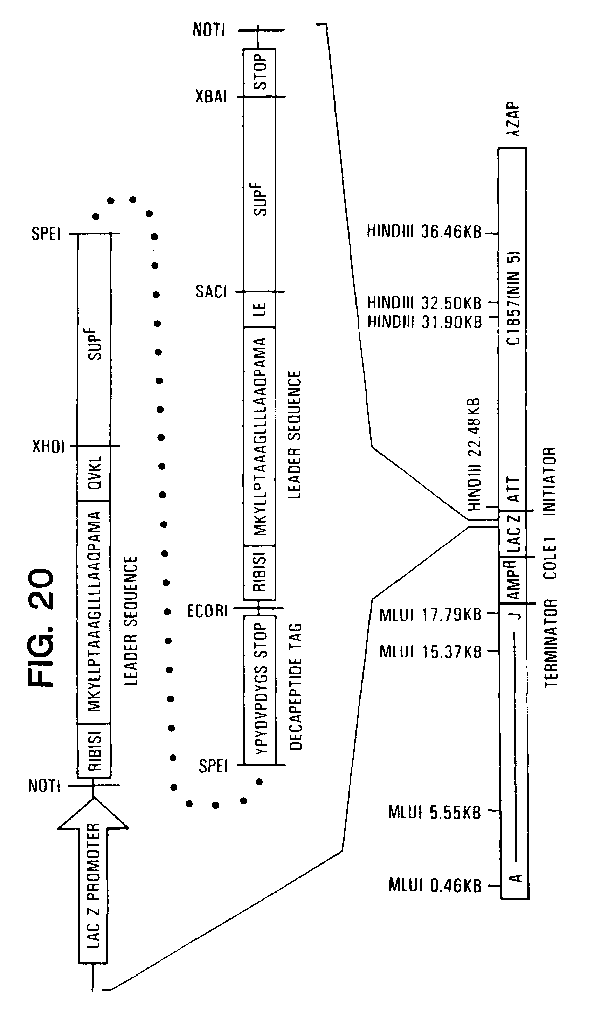

Figure 20 One of the vectors for expression V_{H} and V_{L} in combination are shown. The various essential components of these vectors are shown. The selectable marker (sup F) is shown.

### Detailed Description of the Invention

### A. Definitions

Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. When the nucleoside contains a phosphate group bonded to the 3' or 5' position of the pentose it is referred to as a nucleotide.
Base Pair (bp): a partnership of adenine (A) with thymine (T), or of cytosine (C) with guanine (G) in a double stranded DNA molecule. In RNA, uracil (U) is substituted for thymine.
Nucleic Acid: a polymer of nucleotides, either single or double stranded.
Gene: a nucleic acid whose nucleotide sequence codes for a RNA or polypeptide. A gene can be either RNA or DNA.
Complementary Bases: nucleotides that normally pair up when DNA or RNA adopts a double stranded configuration.
Complementary Nucleotide Sequence: a sequence of nucleotides in a single-stranded molecule of DNA or RNA that is sufficiently complementary to that on another single strand to specifically hybridize to it with consequent hydrogen bonding.
Conserved: a nucleotide sequence is conserved with respect to a preselected (reference) sequence if it non-randomly hybridizes to an exact complement of the preselected sequence.
Hybridization: the pairing of substantially complementary nucleotide sequences (strands of nucleic acid) to form a duplex or heteroduplex by the establishment of hydrogen bonds between complementary base pairs. It is a specific, i.e. non-random, interaction between two complementary polynucleotide that can be competitively inhibited.
Nucleotide Analog: a purine or pyrimidine nucleotide that differs structurally from a, T, G, C, or U, but is sufficiently similar to substitute for the normal nucleotide in a nucleic acid molecule.
DNA Homolog: is a nucleic acid having a preselected conserved nucleotide sequence and a sequence coding for a receptor capable of binding a preselected ligand.
Antibody: The term antibody in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab')₂ and F(v).
Antibody Combining Site: An antibody combining site is that structural portion of an antibody molecule comprised of a heavy and light chain variable and hypervariable regions that specifically binds (immunoreacts with) an antigen. The term immunoreact in its various forms means specific binding between an antigenic determinant-containing molecule and a molecule containing an antibody combining site such as a whole antibody molecule or a portion thereof.
Monoclonal Antibody: The phrase monoclonal antibody in its various grammatical forms refers to a population of antibody molecules that contains only one species of antibody combining site capable of immunoreacting with a particular antigen. A monoclonal antibody thus typically displays a single binding affinity for any antigen with which it immunoreacts. A monoclonal antibody may therefore contain an antibody molecule having a plurality of antibody combining sites, each immunospecific for a different antigen, e.g., a bispecific monoclonal antibody.

### B. Methods

The present invention contemplates a method of isolating from a repertoire of conserved genes a gene coding for a receptor having a preselected catalytic activity.

The conserved gene families is that coding for immunoglobulins.

The immunoglobulins, or antibody molecules, are a large family of molecules that include several types of molecules, such as IgD, IgG, IgA, IgM and IgE. The antibody molecule is typically comprised of two heavy (H) and light (L) chains with both a variable (V) and constant (C) region present on each chain. Several different regions of an immunoglobulin contain conserved sequences useful for isolating an immunoglobulin repertoire. Extensive amino acid and nucleic acid sequence data displaying exemplary conserved sequences is compiled for immunoglobulin molecules by Kabat et al., in Sequences of Proteins of Immunological Interest, National Institutes of Health, Bethesda, MD, 1987.

The C region of the H chain defines the particular immunoglobulin type. Therefore the selection of conserved sequences as defined herein from the C region of the H chain results in the preparation of a repertoire of immunoglobulin genes having members of the immunoglobulin type of the selected C region.

The V region of the H or L chain typically comprises four framework (FR) regions each containing relatively lower degrees of variability that includes lengths of conserved sequences. The use of conserved sequences from the FR1 and FR4 (J region ) framework regions of the V_{H} chain is a preferred exemplary embodiment and is described herein in the Examples. Framework regions are typically conserved across several or all immunoglobulin types and thus conserved sequences contained therein are particularly suited for preparing repertoires having several immunoglobulin types.

The following discussion illustrates the method of the present invention.

Generally, the method combines the following elements:
1. Isolating nucleic acids containing a substantial portion of the immunological repertoire.
2. Preparing polynucleotide primers for cloning polynucleotide segments containing immunoglobulin V_{H} and/or V_{L} region genes.
3. Preparing a gene library containing a plurality of different V_{H} and V_{L} genes from the repertoire.
4. Expressing the V_{H} and/or V_{L} polypeptides in a suitable host, including prokaryotic and eukaryotic hosts, either separately or in the same cell, and either on the same or different expression vectors.
5. Screening the expressed polypeptides for the preselected catalytic activity, and segregating a V_{H}-and/or V_{L}-coding gene identified by the screening process.

A receptor of the subject invention binds a substrate and catalyzes the formation of a product from the substrate. While the topology of the ligand binding site of a catalytic receptor is probably more important for its preselected activity than its affinity (association constant or pKa) for the substrate, the subject catalytic receptors have an association constant for the preselected substrate generally greater than 10³ M⁻¹, more usually greater than 10⁵ M⁻¹ or 10⁶ M⁻¹ and preferably greater than 10⁷ M⁻¹.

Preferably the receptor produced by the subject invention is heterodimeric and is therefore normally comprised of two different polypeptide chains, which together assume a conformation having a binding affinity, or association constant for the preselected ligand that is different, preferably higher, than the affinity or association constant of either of the polypeptides alone, i.e., as monomers. One or both of the different polypeptide chains is derived from the variable region of the light and heavy chains of an immunoglobulin. Typically, polypeptides comprising the light (V_{L}) and heavy (V_{H}) variable regions are employed together for binding the preselected ligand.

A receptor produced by the subject invention can be active in monomeric as well as multimeric forms, either homomeric or heteromeric, preferably heterodimeric. For example, V_{H} and V_{L} ligand binding polypeptide produced by the present invention can be advantageously combined in the heterodimer to modulate the activity of either or to produce an activity unique to the heterodimer. The individual ligand binding polypeptides will be referred to as V_{H} and V_{L} and the heterodimer will be referred to as a Fv.

However, it should be understood that a V_{H} binding polypeptide may contain in addition to the V_{H}, substantially all or a portion of the heavy chain constant region. A V_{L} binding polypeptide may contain, in addition to the V_{L}, substantially all or a portion of the light chain constant region. A heterodimer comprised of a V_{H} binding polypeptide containing a portion of the heavy chain constant region and a V_{L} binding containing substantially all of the light chain constant region is termed a Fab fragment. The production of Fab can be advantageous in some situations because the additional constant region sequences contained in a Fab as compared to a F_{V} could stabilize the V_{H} and V_{L} interaction. Such stabilization could cause the Fab to have higher affinity for antigen. In addition the Fab is more commonly used in the art and thus there are more commercial antibodies available to specifically recognize a Fab.

The individual V_{H} and V_{L} polypeptides will generally have fewer than 125 amino acid residues, more usually fewer than about 120 amino acid residues, while normally having greater than 60 amino acid residues, usually greater than about 95 amino acid residues, more usually greater than about 100 amino acid residues. Preferably, the V_{H} will be from about 110 to about 125 amino acid residues in length while V_{L} will be from about 95 to about 115 amino acid residues in length.

The amino acid residue sequences will vary widely, depending upon the particular idiotype involved. Usually, there will be at least two cysteines separated by from about 60 to 75 amino acid residues and joined by a disulfide bond. The polypeptides produced by the subject invention will normally be substantial copies of idiotypes of the variable regions of the heavy and/or light chains of immunoglobulins, but in some situations a polypeptide may contain random mutations in amino acid residue sequences in order to advantageously improve the desired activity.

In some situations, it is desirable to provide for covalent cross linking of the V_{H} and V_{L} polypeptides, which can be accomplished by providing cysteine resides at the carboxyl termini. The polypeptide will normally be prepared free of the immunoglobulin constant regions, however a small portion of the J region may be included as a result of the advantageous selection of DNA synthesis primers. The D region will normally be included in the transcript of the V_{H}.

In other situations, it is desirable to provide a peptide linker to connect the V_{L} and the V_{H} to form a single-chain antigen-binding protein comprised of a V_{H} and a V_{L}. This single-chain antigen-binding protein would be synthesized as a single protein chain. Such single-chain antigen-binding proteins have been described by Bird et al., Science, 242:423-426 (1988). The design of suitable peptide linker regions is described in U.S. Patent No. 4,704,692 by Robert Landner.

Such a peptide linker could be designed as part of the nucleic acid sequences contained in the expression vector. The nucleic acid sequences coding for the peptide linker would be between the V_{H} and V_{L} DNA homologs and the restriction endonuclease sites used to operatively link the V_{H} an V_{L} DNA homologs to the expression vector.

Such a peptide linker could also be coded for nucleic acid sequences that are part of the polynucleotide primers used to prepare the various gene libraries. The nucleic acid sequence coding for the peptide linker can be made up of nucleic acids attached to one of the primers or the nucleic acid sequence coding for the peptide linker may be derived from nucleic acid sequences that are attached to several polynucleotide primers used to create the gene libraries.

Typically the C terminus region of the V_{H} and V_{L} polypeptides will have a greater variety of the sequences than the N terminus and, based on the present strategy, can be further modified to permit a variation of the normally occurring V_{H} and V_{L} chains. A synthetic polynucleotide can be employed to vary one or more amino in an hypervariable region.

### 1. Isolation Of The Repertoire

To prepare a composition of nucleic acids containing a substantial portion of the immunological gene repertoire, a source of genes coding for the V_{H} and/or V_{L} polypeptides is required. Preferably the source will be a heterogeneous population of antibody producing cells, i.e. B lymphocytes (B cells), preferably rearranged B cells such as those found in the circulation or spleen of a vertebrate. (Rearranged B cells are those in which immunoglobulin gene translocation, i.e., rearrangement, has occurred as evidenced by the presence in the cell of mRNA with the immunoglobulin gene V, D and J region transcripts adjacently located thereon.)

In some cases, it is desirable to bias the repertoire for a preselected activity, such as by using as a source of nucleic acid cells (source cells) from vertebrates in any one of various stages of age, health and immune response. For example, repeated immunization of a healthy animal prior to collecting rearranged B cells results in obtaining a repertoire enriched for genetic material producing a ligand binding polypeptide of high affinity. Conversely, collecting rearranged B cells from a healthy animal whose immune system has not been recently challenged results in producing a repertoire that is not biased towards the production of high affinity V_{H} and/or V_{L} polypeptides.

It should be noted the greater the genetic heterogeneity of the population of cells for which the nucleic acids are obtained, the greater the diversity of the immunological repertoire that will be made available for screening according to the method of the present invention. Thus, cells from different individuals, particularly those having an immunologically significant age difference, and cells from individuals of different strains, races or species can be advantageously combined to increase the heterogeneity of the repertoire.

Thus, in one preferred embodiment, the source cells are obtained from a vertebrate, preferably a mammal, which has been immunized or partially immunized with an antigenic ligand (antigen) against which activity is sought, i.e., a preselected antigen. The immunization can be carried out conventionally. See, Current Protocols in Molecular Biology, Ausubel et al., eds., John Wiley & Sons, NY. Nucleic acids coding for V_{H} and V_{L} polypeptides can be derived from cells producing IgA, IgD, IgE, IgG or IgM, most preferably from IgM and IgG, producing cells.

Methods for preparing fragments of genomic DNA from which immunoglobulin variable region genes can be cloned as a diverse population are well known in the art. See for example Herrmann et al., Methods In Enzymol., 152:180-183, (1987); Frischauf, Methods In Enzymol., 152:183-190 (1987); Frischauf, Methods In Enzymol., 152:190-199 (1987); and DiLella et al., Methods In Enzymol., 152:199-212 (1987). (The teachings of the references cited herein are hereby incorporated by reference.)

The desired gene repertoire can be isolated from either genomic material containing the gene expressing the variable region or the messenger RNA (mRNA) which represents a transcript of the variable region. The difficulty in using the genomic DNA from other than non-rearranged B lymphocytes is in juxtaposing the sequences coding for the variable region, where the sequences are separated by introns. The DNA fragment(s) containing the proper exons must be isolated, the introns excised, and the exons then spliced in the proper order and in the proper orientation. For the most part, this will be difficult, so that the alternative technique employing rearranged B cells will be the method of choice because the C D and J immunoglobulin gene regions have translocated to become adjacent, so that the sequence is continuous (free of introns) for the entire variable regions.

In preferred embodiments, the preparation containing the total cellular mRNA is first enriched for the presence of V_{H} and/or V_{L} coding mRNA. Enrichment is typically accomplished by subjecting the total mRNA preparation or partially purified mRNA product thereof to a primer extension reaction employing a polynucleotide synthesis primer of the present invention.

### 2. Preparation Of Polynucleotide Primers

The term "polynucleotide" as used herein in reference to primers, probes and nucleic acid fragments or segments to be synthesized by primer extension is defined as a molecule comprised of two or more deoxyribonucleotides or ribonucleotides, preferably more than 3. Its exact size will depend on many factors, which in turn depends on the ultimate conditions of use.

The term "primer" as used herein refers to a polynucleotide whether purified from a nucleic acid restriction digest or produced synthetically, which is capable of acting as a point of initiation of synthesis when placed under conditions in which synthesis of a primer extension product which is complementary to a nucleic acid strand is induced, i.e., in the presence of nucleotides and an agent for polymerization such as DNA polymerase, reverse transcriptase and the like, and at a suitable temperature and pH. The primer is preferably single stranded for maximum efficiency, but may alternatively be double stranded. If double stranded, the primer is first treated to separate its strands before being used to prepare extension products. Preferably, the primer is a polydeoxyribonucleotide. The primer must be sufficiently long to prime the synthesis of extension products in the presence of the agents for polymerization. The exact lengths of the primers will depend on may factors, including temperature and the source of primer. For example, depending on the complexity of the target sequence, a polynucleotide primer typically contains 15 to 25 or more nucleotides, although it can contain fewer nucleotides. Short primer molecules generally require cooler temperatures to form sufficiently stable hybrid complexes with template.

The primers used herein are selected to be "substantially" complementary to the different strands of each specific sequence to be synthesized or amplified. This means that the primer must be sufficiently complementary to non-randomly hybridize with its respective template strand. Therefore, the primer sequence may not reflect the exact sequence of the template. For example, a non-complementary nucleotide fragment can be attached to the 5' end of the primer, with the remainder of the primer sequence being substantially complementary to the strand. Such non-complementary fragments typically code for an endonuclease restriction site. Alternatively, non-complementary bases or longer sequences can be interspersed into the primer, provided the primer sequence has sufficient complementarily with the sequence of the strand to be synthesized or amplified to non-randomly hybridize therewith and thereby form an extension product under polynucleotide synthesizing conditions.

The polynucleotide primers can be prepared using any suitable method, such as, for example, the phosphotriester on phosphodiester methods see Narang et al., Meth. Enzymol., 68:90, (1979); U.S. Patent No. 4,356,270; and Brown et al., Meth. Enzymol., 68:109, (1979).

The choice of a primer's nucleotide sequence depends on factors such as the distance on the nucleic acid from the region coding for the desired receptor, its hybridization site on the nucleic acid relative to any second primer to be used, the number of genes in the repertoire it is to hybridize to, and the like.

For example, to produce V_{H}-coding DNA homologs by primer extension, the nucleotide sequence of a primer is selected to hybridize with a plurality of immunoglobulin heavy chain genes at a site substantially adjacent to the V_{H}-coding region so that a nucleotide sequence coding for a functional (capable of binding) polypeptide is obtained. To hybridize to a plurality of different V_{H}-coding nucleic acid strands, the primer must be a substantial complement of a nucleotide sequence conserved among the different strands. Such sites include nucleotide sequences in the constant region, any of the variable region framework regions, preferably the third framework region, leader region, promoter region, J region and the like.

If the V_{H}-coding and V_{L}-coding DNA homologs are to be produced by polymerase chain reaction (PCR) amplification, two primers must be used for each coding strand of nucleic acid to be amplified. The first primer becomes part of the nonsense (minus or complementary) strand and hybridizes to a nucleotide sequence conserved among V_{H} (plus) strands within the repertoire. To produce V_{H} coding DNA homologs, first primers are therefore chosen to hybridize to (i.e. be complementary to) conserved regions within the J region, CH1 region, hinge region, CH2 region, or CH3 region of immunoglobulin genes and the like. To produce a V_{L} coding DNA homolog, first primers are chosen to hybridize with (i.e. be complementary to) a conserved region within the J region or constant region of immunoglobulin light chain genes and the like. Second primers become part of the coding (plus) strand and hybridize to a nucleotide sequence conserved among minus strands. To produce the V_{H}-coding DNA homologs, second primers are therefore chosen to hybridize with a conserved nucleotide sequence at the 5' end of the V_{H}-coding immunoglobulin gene such as in that area coding for the leader or first framework region. It should be noted that in the amplification of both V_{H}- and V_{L}-coding DNA homologs the conserved 5' nucleotide sequence of the second primer can be complementary to a sequence exogenously added using terminal deoxynucleotidyl transferase as described by Loh et al., Sci. Vol 243:217-220 (1989). One or both of the first and second primers can contain a nucleotide sequence defining an endonuclease recognition site. The site can be heterologous to the immunoglobulin gene being amplified and typically appears at or near the 5' end of the primer.

Primers of the present invention may also contain a DNA-dependent RNA polymerase promoter sequence or its complement. See for example, Krieg et al.,Nucleic Acids Research, 12:7057-70 (1984); Studier et al., J. Mol. Biol., 189:113-130 (1986); and Molecular Cloning: A Laboratory Manual, Second Edition, Maniatis et al., eds., Cold Spring Harbor, NY (1989).

### 3. Preparing a Gene Library

The strategy used for cloning, i.e., substantially reproducing, the V_{H} and/or V_{L} genes contained within the isolated repertoire will depend, as is well known in the art, on the type, complexity, and purity of the nucleic acids making up the repertoire. Other factors include whether or not the genes are to be amplified and/or mutagenized.

In one strategy, the object is to clone the V_{H}- and/or V_{L}-coding genes from a repertoire comprised of polynucleotide coding strands, such as mRNA and/or the sense strand of genomic DNA.

The first primer extension is performed by mixing the first primer, preferably a predetermined amount thereof, with the nucleic acids of the repertoire, preferably a predetermined amount thereof, to form a first primer extension reaction admixture. The admixture is maintained under polynucleotide synthesizing conditions for a time period, which is typically predetermined, sufficient for the formation of a first primer extension reaction product, thereby producing a plurality of different V_{H}-coding DNA homolog complements. The complements are then subjected to a second primer extension reaction by treating them with a second polynucleotide synthesis primer having a preselected nucleotide sequence, to form a second primer extension reaction admixture. The admixture is maintained under polynucleotide synthesizing conditions for a time period, which is typically predetermined, sufficient for the formation of a first primer extension reaction product, thereby producing a gene library containing a plurality of different V_{H}-and/or V_{L}-coding DNA homologs.

A plurality of first primer and/or a plurality of second primers can be used in each amplification, or an individual pair of first and second primers can be used. In any case, the amplification products of amplifications using the same or different combinations of first and second primers can be combined to increase the diversity of the gene library.

In another strategy, the object is to clone the V_{H}- and/or V_{L}-coding genes from a repertoire by providing a polynucleotide complement of the repertoire, such as the anti-sense strand of genomic dsDNA or the polynucleotide produced by subjecting mRNA to a reverse transcriptase reaction. Methods for producing such complements are well known in the art.

The primer extension reaction is performed using any suitable method.

The newly synthesized strand and its complementary nucleic acid strand form a double-stranded molecule which can be used in the succeeding steps of the process.

The first and/or second primer extension reaction discussed above can advantageously be used to incorporate into the receptor a preselected epitope useful in immunologically detecting and/or isolating a receptor. This is accomplished by utilizing a first and/or second polynucleotide synthesis primer or expression vector to incorporate a predetermined amino acid residue sequence into the amino acid residue sequence of the receptor.

After producing V_{H}- and/or V_{L}-coding DNA homologs for a plurality of different V_{H}- and/or V_{L}-coding genes within the repertoire, the homologs are typically amplified. It is preferred to first amplify the DNA homologs by subjecting them to a polymerase chain reaction (PCR) prior to inserting them into a vector. In fact, in preferred strategies, the first and/or second primer extension reactions used to produce the gene library are the first and second primer extension reactions in a polymerase chain reaction.

In preferred embodiments only one pair of first and second primers is used per amplification reaction. The amplification reaction products obtained from a plurality of different amplifications, each using a plurality of different primer pairs, are then combined.

However, the present invention also contemplates DNA homolog production via co-amplification (using two pairs of primers), and multiplex amplification (using up to about 8, 9 or 10 primer pairs).

The V_{H}- and V_{L}-coding DNA homologs produced by PCR amplification are typically in double-stranded form and have contiguous or adjacent to each of their termini a nucleotide sequence defining an endonuclease restriction site. Digestion of the V_{H}- and V_{L}-coding DNA homologs having restriction sites at or near their termini with one or more appropriate endonucleases results in the production of homologs having cohesive termini of predetermined specificity.

In preferred embodiments, the PCR process is used not only to amplify the V_{H}- and/or V_{L}-coding DNA homologs of the library, but also to induce mutations within the library and thereby provide a library having a greater heterogeneity. First, it should be noted that the PCR processes itself is inherently mutagenic due to a variety of factors well known in the art. Second, in addition to the mutation inducing variations described in the above referenced U.S. Patent No. 4,683,195, other mutation inducing PCR variations can be employed. For example, the PCR reaction admixture, i.e., the combined first and second primer extension reaction admixtures, can be formed with different amounts of one or more of the nucleotides to be incorporated into the extension product. Under such conditions, the PCR reaction proceeds to produce nucleotide substitutions within the extension product as a result of the scarcity of a particular base. Similarly, approximately equal molar amounts of the nucleotides can be incorporated into the initial PCR reaction admixture in an amount to efficiently perform X number of cycles, and then cycling the admixture through a number of cycles in excess of X, such as, for instance, 2X. Alternatively, mutations can be induced during the PCR reaction by incorporating into the reaction admixture nucleotide derivatives such as inosine, not normally found in the nucleic acids of the repertoire being amplified. During subsequent in vivo amplification, the nucleotide derivative will be replaced with a substitute nucleotide thereby inducing a point mutation.

### 4. Expressing the V_{H} and/or V_{L} DNA Homologs.

The V_{H-} and/or V_{L}-coding DNA homologs contained within the library produced by the above-described method can be operatively linked to a vector for amplification and/or expression.

Preferred vectors are those capable of autonomous replication and/or expression of nucleic acids to which they are linked. Vectors capable of directing the expression of genes to which they are operatively linked are referred to herein as "expression vectors".

The choice of vector to which a V_{H}- and/or V_{L}-coding DNA homolog is operatively linked depends directly, as is well known in the art, on the functional properties desired, e.g., replication or protein expression, and the host cell to be transformed, these being limitations inherent in the art of constructing recombinant DNA molecules.

In preferred embodiments, the vector utilized includes a prokaryotic replicon. In addition, those embodiments that include a prokaryotic replicon also include a gene whose expression confers a selective advantage, such as drug resistance, to a bacterial host transformed therewith. Typical bacterial drug resistance genes are those that confer resistance to ampicillin or tetracycline.

Those vectors that include a prokaryotic replicon can also include a prokaryotic promoter capable of directing the expression (transcription and translation) of the V_{H}- and/or V_{L}-coding homologs in a bacterial host cell, such as E. coli transformed therewith. Typical vector plasmids are pUC8, pUC9, pBR322, and pBR329 available from BioRad Laboratories, (Richmond, CA) and pPL and pKK223 available from Pharmacia, (Piscataway, NJ).

Expression vectors compatible with eukaryotic cells, preferably those compatible with vertebrate cells, can also be used. Eukaryotic cell expression vectors are well known in the art and are available from several commercial sources. Typically, such vectors are provided containing convenient restriction sites for insertion of the desired DNA homolog. Typical of such vectors are pSVL and pKSV-10 (Pharmacia), pBPV-1/PML2d (International Biotechnologies, Inc.), and pTDT1 (ATCC, No. 31255).

In preferred embodiments, the eukaryotic cell expression vectors used include a selection marker that is effective in an eukaryotic cell, preferably a drug resistant selection marker. A preferred drug resistance marker is the gene whose expression results in neomycin resistance, i.e., the neomycin phosphotransferase (neo) gene. Southern et al., J. Mol. Appl. Genet., 1:327-341 (1982).

The use of retroviral expression vectors to express the genes of the V_{H} and/or V_{L}-coding DNA homologs is also contemplated, preferably one which is replication-incompetent in eukaryotic cells. The construction and use of retroviral vectors has been described by Sorge et al., Mol. Cel. Biol., 4:1730-1737 (1984).

In preferred embodiments, the V_{H}-coding and V_{L}-coding DNA homologs of diverse libraries are randomly combined in vitro for polycistronic expression from individual vectors. Random combination in vitro can be accomplished using two expression vectors distinguished from one another by the location on each of a restriction site common to both. Preferably the vectors are linear double stranded DNA, such as a Lambda Zap derived vector as described herein. In the first vector, the site is located between a promoter and a polylinker, i.e., 5' terminal (upstream relative to the direction of expression) to the polylinker but 3' terminal (downstream relative to the direction of expression). In the second vector, the polylinker is located between a promoter and the restriction site, i.e., the restriction site is located 3' terminal to the polylinker, and the polylinker is located 3' terminal to the promoter.

In preferred embodiments, each of the vectors defines a nucleotide sequence coding for a ribosome binding and a leader, the sequence being located between the promoter and the polylinker, but downstream (3' terminal) from the shared restriction site if that site is between the promoter and polylinker. Also preferred are vectors containing a stop codon downstream from the polylinker, but upstream from any shared restriction site if that site is downstream from the polylinker. The first and/or second vector can also define a nucleotide sequence coding for a peptide tag. The tag sequence is typically located downstream from the polylinker but upstream from any stop codon that may be present. In preferred embodiments, the vectors contain selectable markers such that the presence of a portion of that vector, i.e. a particular lambda arm, can be selected for or selected against. Typical selectable markers are well known to those skilled in the art. Examples of such markers are antibiotic resistance genes, genetically selectable markers, mutation suppressors such as amber suppressors and the like. The selectable markers are typically located upstream of the promoter and/or downstream of the second restriction site. In preferred embodiments, one selectable marker is located upstream of the promoter on the first vector containing the V_{H}-coding DNA homologs. A second selectable marker is located downstream of the second restriction site on the vector containing the V_{L}-coding DNA homologs. This second selectable marker may be the same or different from the first as long as when the V_{H}-coding vectors and the V_{L}-coding vectors are randomly combined via the first restriction site the resulting vectors containing both V_{H} and V_{L} and both selectable markers can be selected.

Random combination is accomplished by ligating V_{H}-coding DNA homologs into the first vector, typically at a restriction site or sites within the polylinker. Similarly, V_{L}-coding DNA homologs are ligated into the second vector, thereby creating two diverse populations of expression vectors. It does not matter which type of DNA homolog, i.e., V_{H} or V_{L}, is ligated to which vector, but it is preferred, for example, that all V_{H}-coding DNA homologs are ligated to either the first or second vector, and all of the V_{L}-coding DNA homologs are ligated to the other of the first or second vector. The members of both populations are then cleaved with an endonuclease at the shared restriction site, typically by digesting both populations with the same enzyme. The resulting product is two diverse populations of restriction fragments where the members of one have cohesive termini complementary to the cohesive termini of the members of the other. The restriction fragments of the two populations are randomly ligated to one another, i.e., a random, interpopulation ligation is performed, to produce a diverse population of vectors each having a V_{H}-coding and V_{L}-coding DNA homolog located in the same reading frame and under the control of second vector's promoter. Of course, subsequent recombinations can be effected through cleavage at the shared restriction site, which is typically reformed upon ligation of members from the two populations, followed by subsequent religations.

The resulting construct is then introduced into an appropriate host to provide amplification and/or expression of the V_{H}- and/or V_{L}-coding DNA homologs, either separately or in combination. When coexpressed within the same organism, either on the same or the different vectors, a functionally active Fv is produced. When the V_{H} and V_{L} polypeptides are expressed in different organisms, the respective polypeptides are isolated and then combined in an appropriate medium to form a Fv.

The host cell can be either prokaryotic or eukaryotic. Bacterial cells are preferred prokaryotic host cells and typically are a strain of E. coli such as, for example, the E. coli strain DH5 available from Bethesda Research Laboratories, Inc., Bethesda, MD. Preferred eukaryotic host cells include yeast and mammalian cells, preferably vertebrate cells such as those from a mouse, rat, monkey or human cell line.

Transformation of appropriate cell hosts with a recombinant DNA molecule of the present invention is accomplished by methods that typically depend on the type of vector used. With regard to transformation of prokaryotic host cells, see, for example, Cohen et al., Proc. Natl. Acad. Sci., USA, 69:2110 (1972); and Maniatis et al., Molecular Cloning: A Laboratory Manual, Cold Spring Harbor, NY (1982). With regard to the transformation of vertebrate cells with retroviral vectors containing rDNAs, see for example, Sorge et al., Mol. Cell. Biol., 4:1730-1737 (1984); Graham et al., Virol., 52:456 (1973); and Wigler et al., Proc. Natl. Acad. Sci., USA, 76:1373-1376 (1979).

### 5. Screening For Expression of V_{H} and/or V_{L} Polypeptides

Successfully transformed cells, i.e., cells containing a V_{H}- and/or V_{L}-coding DNA homolog operatively linked to a vector, can be identified by any suitable well known technique for detecting the binding of a receptor to a ligand or the presence of a polynucleotide coding for the receptor, preferably its active site. Preferred screening assays are those where the binding of ligand by the receptor produces a detectable signal, either directly or indirectly. Such signals include, for example, the production of a complex, formation of a catalytic reaction product, the release or uptake of energy, and the like.

In addition to directly assaying for the presence of a V_{H}- and/or V_{L}-coding DNA homolog, successful transformation can be confirmed by well known immunological methods, especially when the V_{H} and/or V_{L} polypeptides produced contain a preselected epitope.

### D. Expression Vectors

The present invention also contemplates various expression vectors useful in performing, inter alia, the methods of the present invention. Each of the expression vectors is a novel derivative of Lambda Zap.

### 1. Lambda Zap II

Lambda Zap II is prepared by replacing the Lambda S gene of the vector Lambda Zap with the Lambda S gene from the Lambda gt10 vector, as described in Example 6.

### 2. Lambda Zap II V_{H}

Lambda Zap II V_{H} is prepared by inserting the synthetic DNA sequences illustrated in Figure 6A into the above-described Lambda Zap II vector. The inserted nucleotide sequence advantageously provides a ribosome binding site (Shine-Dalgarno sequence) to permit proper imitation of mRNA translation into protein, and a leader sequence to efficiently direct the translated protein to the periplasm. The preparation of Lambda Zap II V_{H} is described in more detail in Example 9, and its features illustrated in Figures 6A and 7.

### 3. Lambda Zap II V_{L}

Lambda Zap II V_{L} is prepared as described in Example 12 by inserting into Lambda Zap II the synthetic DNA sequence illustrated in Figure 6B. Important features of Lambda Zap II V_{L} are illustrated in Figure 8.

### 4. Lambda Zap II V_{L} II

Lambda Zap II V_{L} II is prepared as described in Example 11 by inserting into Lambda Zap II the synthetic DNA sequence illustrated in Figure 10.

The above-described vectors are compatible with E. coli hosts, i.e., they can express for secretion into the periplasm proteins coded for by genes to which they have been operatively linked for expression.

### Examples

The following examples are intended to illustrate, but not limit, the scope of the invention.

### 1. Polynucleotide Selection

The nucleotide sequences encoding the immunoglobulin protein CDR's are highly variable. However, there are several regions of conserved sequences that flank the V_{H} domains. For instance, contain substantially conserved nucleotide sequences, i.e., sequences that will hybridize to the same primer sequence. Therefore, polynucleotide synthesis (amplification) primers that hybridize to the conserved sequences and incorporate restriction sites into the DNA homolog produced that are suitable for operatively linking the synthesized DNA fragments to a vector were constructed. More specifically, the DNA homologs were inserted into Lambda ZAP II vector (Stratagene Cloning System, La Jolla, CA) at the Xho I and EcoR I sites. For amplification of the V_{H} domains, the 3' primer (primer 12 in Table 1), was designed to be complementary to the mRNA in the J_{H} region. In all cases, the 5' primers (primers 1-10, Table 1) were chosen to be complementary to the first strand cDNA in the conserved N-terminus region (antisense strand). Initially amplification was performed with a mixture of 32 primers (primer 1, Table 1) that were degenerate at five positions. Hybridoma mRNA could be amplified with mixed primers, but initial attempts to amplify mRNA from spleen yielded variable results. Therefore, several alternatives to amplification using the mixed 5' primers were compared.

The first alternative was to construct multiple unique primers, eight of which are shown in Table 1, corresponding to individual members of the mixed primer pool. The individual primers 2-9 of Table 1 were constructed by incorporating either of the two possible nucleotides at three of the five degenerate positions.

The second alternative was to construct a primer containing inosine (primer 10, Table 1) at four of the variable positions based on the published work of Takahashi, et al., Proc. Natl. Acad. Sci. (U.S.A.), 82:1931-1935, (1985) and Ohtsuka et al., J. Biol. Chem., 260: 2605-2608, (1985). This primer has the advantage that it is not degenerate and, at the sane time minimizes the negative effects of mismatches at the unconserved positions as discussed by Martin et al., Nuc. Acids Res., 13:8927 (1985). However, it was not known if the presence of inosine nucleotides would result in incorporation of unwanted sequences in the cloned V_{H} regions. Therefore, inosine was not included at the one position that remains in the amplified fragments after the cleavage of the restriction sites. As a result, inosine was not in the cloned insert.

Additional V_{H} amplification primers including the unique 3' primer were designed to be complementary to a portion of the first constant region domain of the gamma 1 heavy chain mRNA (primers 15 and 16, Table 1). These primers will produce DNA homologs containing polynucleotides coding for amino acids from the V_{H} and the first constant region domains of the heavy chain. These DNA homologs can therefore be used to produce Fab fragments rather than an F_{V}.

Additional unique 3' primers designed to hybridize to similar regions of another class of immunoglobulin heavy chain such as IgM, IgE and IgA are contemplated. Over 3' primers that hybridize to a specific region of a specific class of CH₁ constant region and are adapted for transferring the V_{H} domains amplified using this primer to an expression vector capable of expressing those V_{H} domains with a different class of heavy or light chain constant region is also contemplated.

As a control for amplification from spleen or hybridoma mRNA, a set of primers hybridizing to a highly conserved region within the constant region IgG, heavy chain gene were constructed. The 5' primer (primer 11, Table 1) is complementary to the cDNA in the C_{H}2 region whereas the 3' primer (primer 13, Table 1) is complementary to the mRNA in the C_{H}3 region. It is believed that no mismatches were present between these primers and their templates.

The nucleotide sequences encoding the V_{L} CDRs are highly variable. However, there are several regions of conserved sequences that flank the V_{L} CDR domains including the J_{L}, V_{L} framework regions and V_{L} leader/promotor. Therefore, amplification primers that hybridize to the conserved sequences and incorporate restriction sites that allow cloning the amplified fragments into the pBluescript SK- vector cut with Nco I and Spe I were constructed. For amplification of the V_{L} CDR domains, the 3' primer (primer 14 in Table 1), was designed to be complementary to the mRNA in the J_{L} regions. The 5' primer (primer 15, Table 1) was chosen to be complementary to the first strand cDNA in the conserved N-terminus region (antisense strand).

A second set of amplification primers for amplification of the V_{L} CDR domains the 5' primers (primers 1-8 in Table 2) were designed to be complementary to the first strand cDNA in the conserved N-terminus region. These primers also introduced a Sac I restriction endonuclease site to allow the V_{L} DNA homolog to be cloned into the V_{L}II-expression vector. The 3' V_{L} amplification primer (primer 9 in Table 2) was designed to be complementary to the mRNA in the J_{L} regions and to introduce the Xba I restriction endonuclease site required to insert the V_{L} DNA homolog into the V_{L}II-expression vector (Figure a).

Additional 3' V_{L} amplification primers were designed to hybridize to the constant region of either kappa or lambda mRNA (primers 10 and 11 in Table 2). These primers allow a DNA homolog to be produced containing polynucleotide sequences coding for constant region amino acids of either kappa or lambda chain. These primers make it possible to produce an Fab fragment rather than an F_{V}.

The primers used for amplification of kappa light chain sequences for construction of Fabs are shown at least in Table 2. Amplification with these primers was performed in 5 separate reactions, each containing one of the 5' primers (primers 3-6, and 12) and one of the 3' primers (primer 13). The remaining 3' primer (primer 9) has been used to construct Fᵥ, fragments. The 5' primers contain a Sac I restriction site and the 3' primers contain a Xba I restriction site.

The primers used for amplification of heavy chain Fd fragments for construction of Fabs are shown at least in Table 1. Amplification was performed in eight separate reactions, each containing one of the 5' primers (primers 2-9) and one of the 3' primers (primer 15). The remaining 5' primers that have been used for amplification in a single reaction are either a degenerate primer (primer 1) or a primer that incorporates inosine at four degenerate positions (primer 10, Table 1, and primers 17 and 18, Table 2). The remaining 3' primer (primer 14, Table 2) has been used to construct Fᵥ fragments. Many of the 5' primers incorporate a Xho I site, and the 3' primers incorporate a Spe I restriction site.

V_{H} amplification primers designed to amplify human heavy chain variable regions are shown in Table 2. One of the 5' heavy chain primer contains inosine residues at degenerate nucleotide positions allowing a single primer to hybridize to a large number of variable region sequences. Primers designed to hybridize to the constant region sequences of various IgG mRNAs are also shown in Table 2.

V_{L} amplification primers designed to amplify human light chain variable regions of both the lambda and kappa isotypes are also shown in Table 2.

All primers and synthetic polynucleotides used herein and shown on Tables 1-4 were either purchased from Research Genetics in Huntsville, Alabama or synthesized on an Applied Biosystems DNA synthesizer, model 381A, using the manufacturer's instruction.

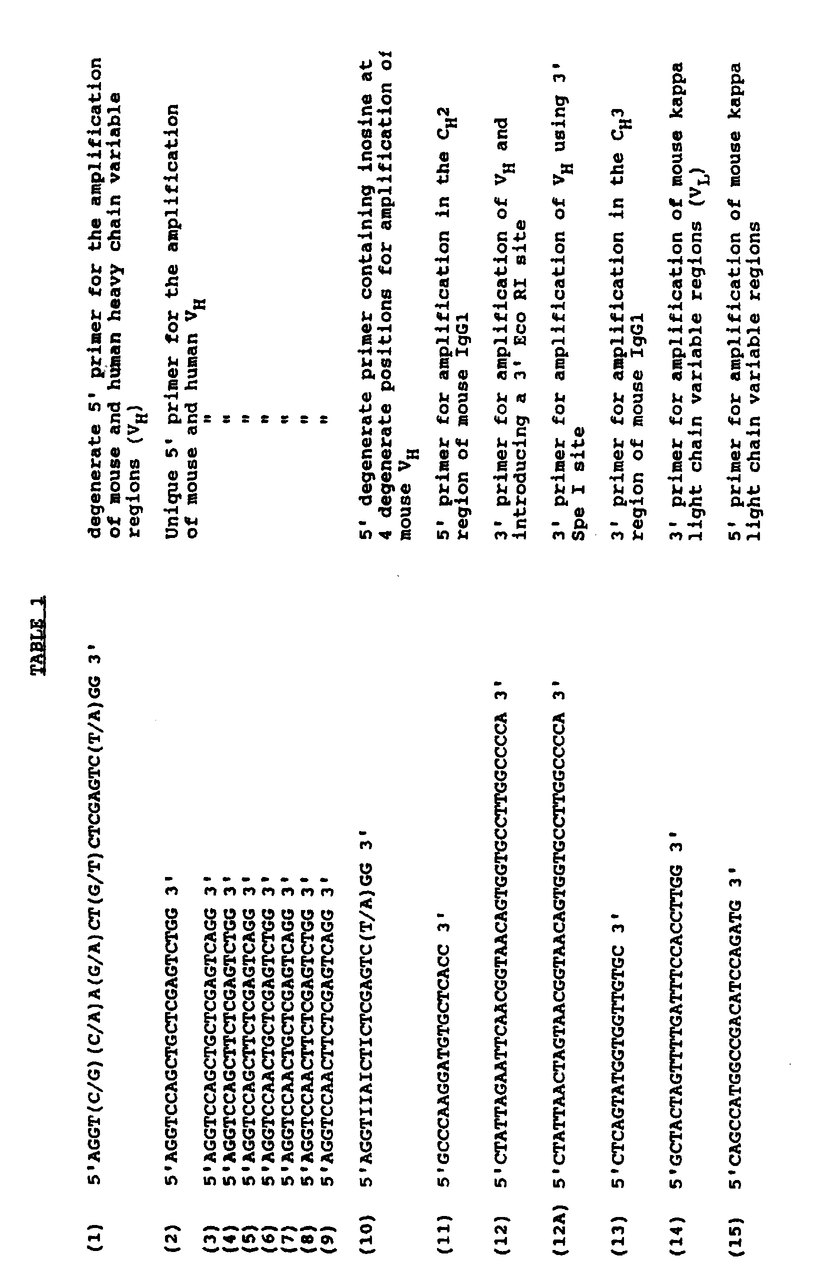

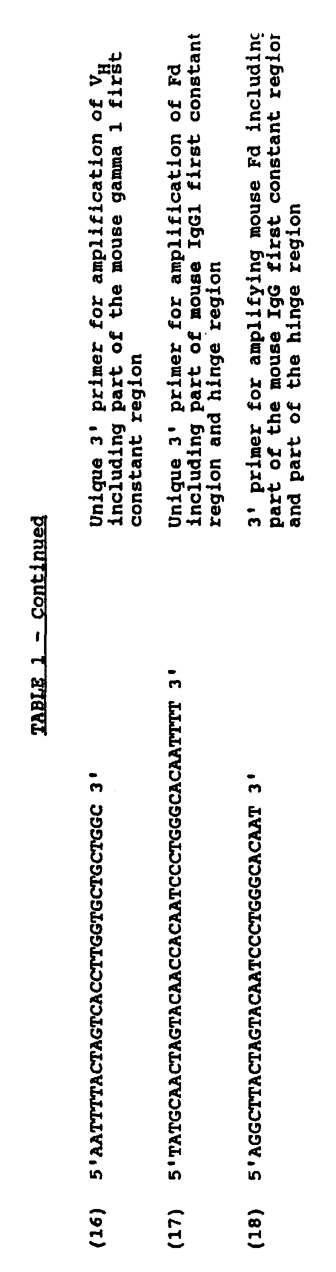

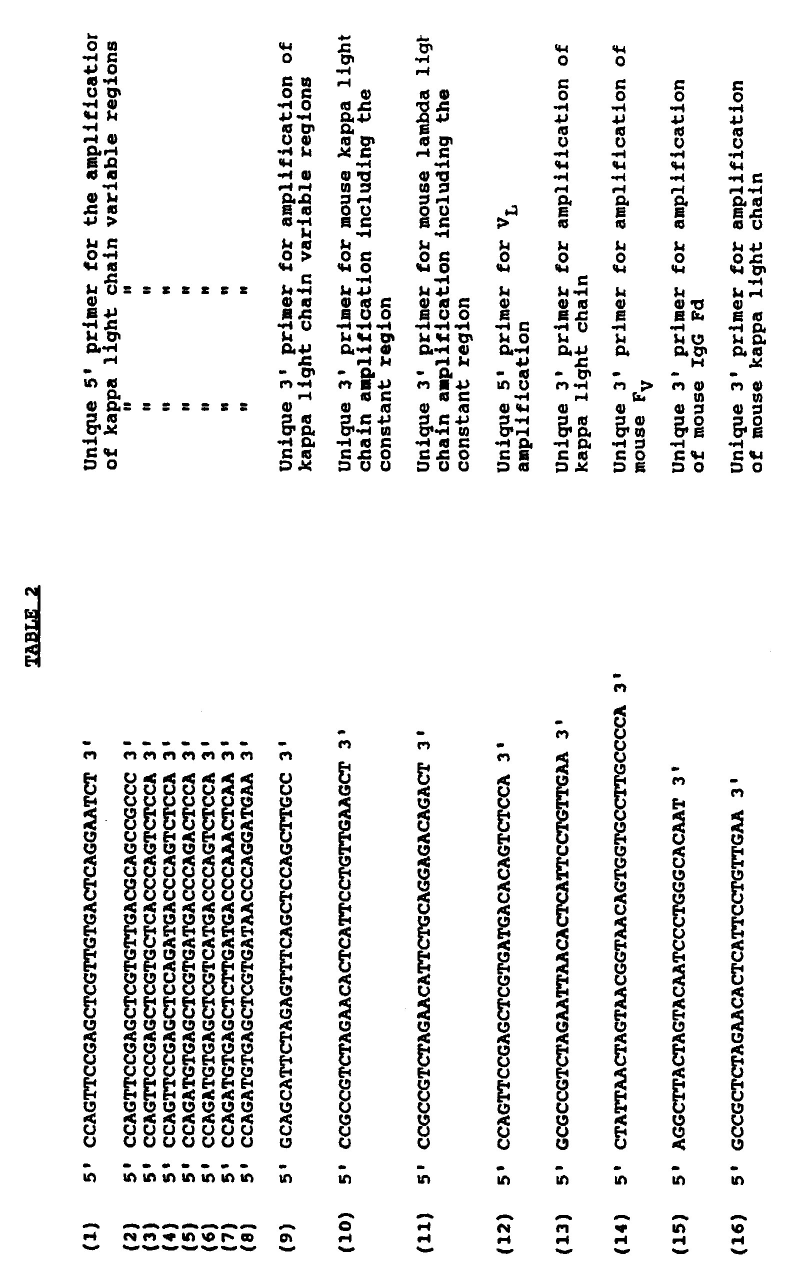

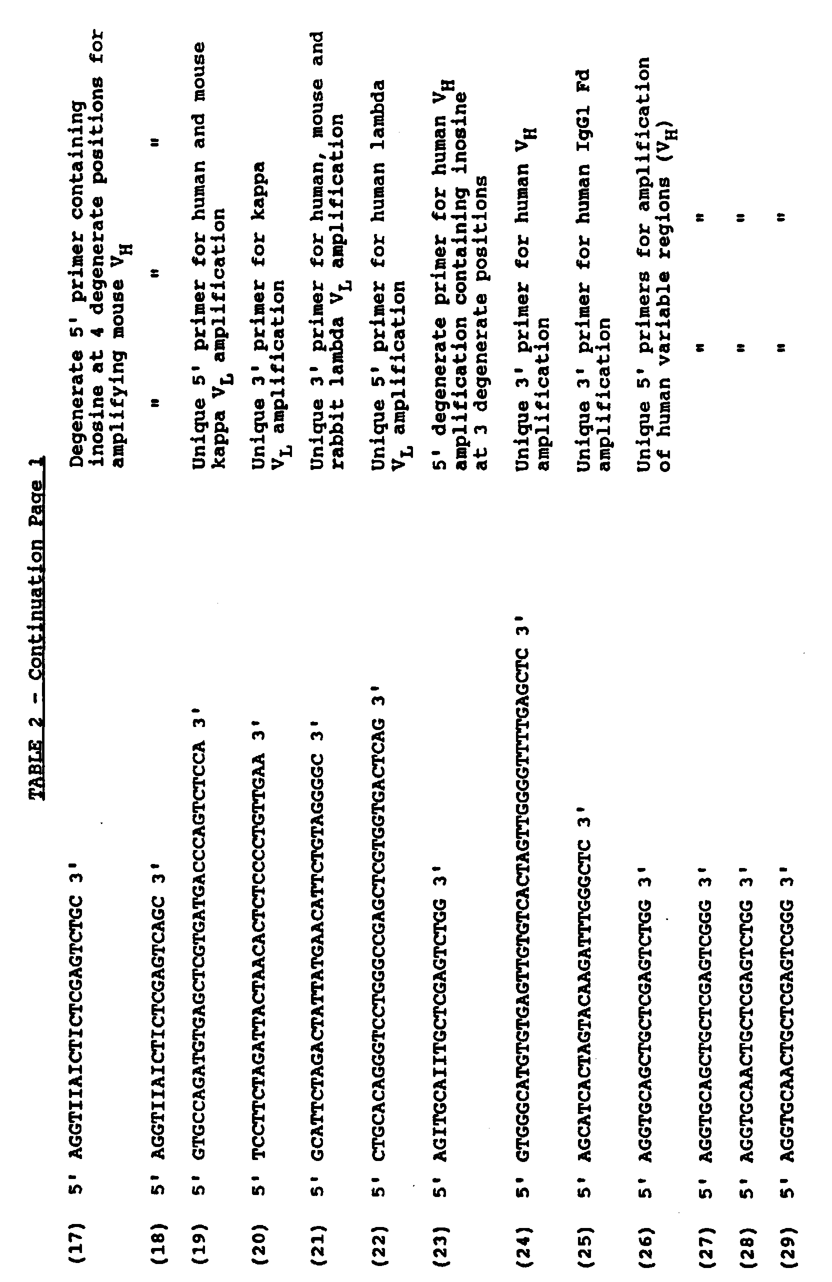

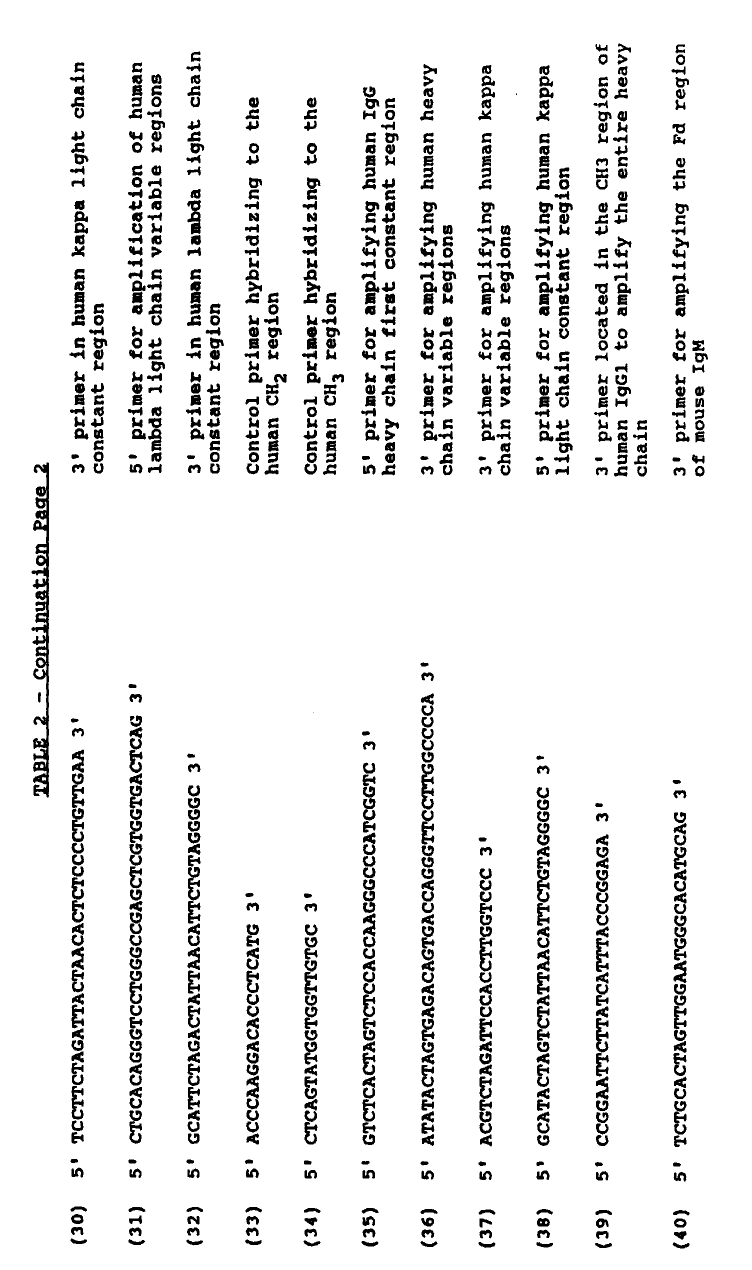

### 2. Production Of A V_{H} Coding Repertoire Enriched In FITC Binding Proteins

Fluorescein isothiocyanate (FITC) was selected as a ligand for receptor binding. It was further decided to enrich by immunization the immunological gene repertoire, i.e., V_{H}- and Vₗ-coding gene repertoires, for genes coding for anti-FITC receptors. This was accomplished by linking FITC to keyhole limpet hemocyanin (KLH) using the techniques described in Antibodies A Laboratory Manual, Harlow and Lowe, eds., Cold Spring Harbor, NY, (1988).

The KLH-FITC conjugate was prepared for injection into mice by adding 100 µg of the conjugate to 250 µl of phosphate buffered saline (PBS). An equal volume of complete Freund's adjuvant was added and emulsified in the entire solution for 5 minutes. A 129 G_{IX+} mouse was injected with 300 µl of the emulsion, and boosted at two weeks and one month. Five days after This final injection the mice were sacrificed and total cellular RNA was isolated from their spleens.

Hybridoma PCP 8D11 producing an antibody immunospecific for phosphonate ester was cultured in DMEM media (Gibco Laboratories, Grand Island, NY) containing 10 percent fetal calf serum supplemented with penicillin and streptomycin. About 5 x 10⁸ hybridoma cells were harvested and washed twice in phosphate buffered saline. Total cellular RNA was prepared from these isolated hybridoma cells.

### 3. Preparation Of A V_{H}-Coding Gene Repertoire

Total cellular RNA was prepared from the spleen of a single mouse immunized with KLH-FITC as described in Example 2 using the RNA preparation methods described by Chomczynski et al., Anal Biochem., 162:156-159 (1987)using the manufacturer's instructions and the RNA isolation kit produced by Stratagene Cloning Systems, La Jolla, CA.

Messenger RNA (mRNA) enriched for sequences containing long poly A tracts was prepared from the total cellular RNA using methods described in Molecular Cloning A Laboratory Manual, Maniatias et al., eds., Cold Spring Harbor, NY, (1982).

The messenger RNA isolated by the above process contains a plurality of different V_{H} coding polynucleotides, i.e., greater than about 10⁴ different V_{H}-coding genes.

### 4. Preparation Of A Single V_{H} Coding Polynucleotide

Polynucleotides coding for a single V_{H} were isolated according to Example 3 except total cellular RNA was extracted from monoclonal hybridoma cells prepared in Example 2. The polynucleotides isolated in this manner code for a single V_{H}.

### 5. DNA Homolog Preparation

In preparation for PCR amplification, mRNA prepared according to the above examples was used as a template for cDNA synthesis by a primer extension reaction. In a typical 50 µl transcription reaction, 5-10 ug of spleen or hybridoma mRNA in water was first hybridized (annealed) with 500 ng (50.0 pmol) of the 3' V_{H} primer (primer 12, Table 1), at 65C for five minutes. Subsequently, the mixture was adjusted to 1.5 mM dATP, dCTP, dGTP and dTTP, 40 mM Tris-HCl at pH 8.0, 8 mM MgCl₂, 50 mM NaCl, and 2 mM spermidine. Moloney-Murine Leukemia virus Reverse transcriptase (Stratagene Cloning Systems), 26 units, was added and the solution was maintained for 1 hour at 37C.

PCR amplification was performed in a 100 µl reaction containing the products of the reverse transcription reaction (approximately 5 ug of the cDNA/RNA hybrid), 300 ng of 3' V_{H} primer (primer 12 of Table 1), 300 ng each of the 5' V_{H} primers (primer 2-10 of Table 1) 200 mM of a mixture of dNTP's, 50 mM KCl, 10 mM Tris-HCl pH 8.3, 15 mM MgCl₂, 0.1% gelatin and 2 units of Tag DNA polymerase. The reaction mixture was overlaid with mineral oil and subjected to 40 cycles of amplification. Each amplification cycle involved denaturation at 92C for 1 minute, annealing at 52C for 2 minutes and polynucleotide synthesis by Primer extension (elongation) at 72C for 1.5 minutes. The amplified V_{H}-coding DNA homolog containing samples were extracted twice with phenol/chloroform, once with chloroform, ethanol precipitated and were stored at -70C in 10 mM Tris-HCl, (pH, 7.5) and 1 mM EDTA.

Using unique 5' primers (2-9, Table 1), efficient V_{H}-coding DNA homolog synthesis and amplification from the spleen mRNA was achieved as shown in Figure 3, lanes R17-R24. The amplified cDNA (V_{H}-coding DNA homolog) is seen as a major band of the expected size (360 bp). The intensities of the amplified V_{H}-coding polynucleotide fragment in each reaction appear to be similar, indicating that all of these primers are about equally efficient in initiating amplification. The yield and quality of the amplification with these primers was reproducible.

The primer containing inosine also synthesized amplified V_{H}-coding DNA homologs from spleen mRNA reproducibly, leading to the production of the expected sized fragment, of an intensity similar to that of the other amplified cDNAs (Figure 4, Lane R16). This result indicated that the presence of inosine also permits efficient DNA homolog synthesis and amplification, clearly indicating how useful such primers are in generating a plurality of V_{H}-coding DNA homologs. Amplification products obtained from the constant region primers (primers 11 and 13, Table 1) were more intense indicating that amplification was more efficient, possibly because of a higher degree of homology between the template and primers (Figure 4, Lane R9). Based on these results, a V_{H}-coding gene library was constructed from the products of eight amplifications, each performed with a different 5' primer. Equal portions of the products from each primer extension reaction were mixed and the mixed product was then used to generate a library of V_{H}-coding DNA homolog-containing vectors.

DNA homologs of the V_{L} were prepared from the purified mRNA prepared as described above. In preparation for PCR amplification, mRNA prepared according to the above examples was used as a template for cDNA synthesis. In a typical 50 µl transcription reaction, 5-10 ug of spleen or hybridoma mRNA in water was first annealed with 300 ng (50.0 pmol) of the 3' V_{L} primer (primer 14, Table 1), at 65°C for five minutes. Subsequently,the mixture was adjusted to 1.5 mM dATP, dCTP, dGTP, and dTTP, 40 mM Tris-HCl at pH 8.0, 8 mM MgCl₂, 50 mM NaCl, and 2 mM spermidine. Moloney-Murine Leukemia virus reverse transcriptase (Stratagene Cloning Systems), 26 units, was added and the solution was maintained for 1 hour at 37°C. The PCR amplification was performed in a 100 µl reaction containing approximately 5 µg of the cDNA/RNA hybrid produced as described above, 300 ng of the 3' V_{L} primer (primer 14 of Table 1), 300 ng of the 5' V_{L} primer (primer 15 of Table 1), 200 mM of a mixture of dNTP's, 50 mM KC1, 10 mM Tris-HCl pH 8.3, 15 mM MgCl₂, 0.1% gelatin and 2 units of Tag DNA polymerase. The reaction mixture was overlaid with mineral oil and subjected to 40 cycles of amplification. Each amplification cycle involved denaturation at 92°C for 1 minute, annealing at 52°C for 2 minutes and elongation at 72°C for 1.5 minutes. The amplified samples were extracted twice with phenol/chloroform, once with chloroform, ethanol precipitated and were stored at -70°C in 10 mM Tris-HCl at 7.5 and 1 mM EDTA.

### 6. Inserting DNA Homologs Into Vectors

In preparation for cloning a library enriched in V_{H} sequences, PCR amplified products (2.5 mg/30 µl of 150 mM NaCl, 8 mM Tris-HCl (pH 7.5), 6 mM MgSO₄, 1 mM DTT, 200 mg/ml bovine serum albumin (BSA) at 37C were digested with restriction enzymes Xho I (125 units) and EcoR I (10 U) and purified on a 1% agarose gel. In cloning experiments which required a mixture of the products of the amplification reactions, equal volumes (50 µl, 1-10 ug concentration) of each reaction mixture were combined after amplification but before restriction digestion. After gel electrophoresis of the digested PCR amplified spleen mRNA, the region of the gel containing DNA fragments of approximately 350 bps was excised, electro-eluted into a dialysis membrane, ethanol precipitated and re-suspended in 10 mM Tris-HCl pH 7.5 and 1 mM EDTA to a final concentration of 10 ng/µl. Equimolar amounts of the insert were then ligated overnight at 5°C to 1 µg of Lambda ZAP™ II vector (Stratagene Cloning Systems, La Jolla, CA) previously cut by EcoR I and Xho I. A portion of the ligation mixture (1 µl) was packaged for 2 hours at room temperature using Gigapack Gold packaging extract (Stratagene Cloning Systems, La Jolla, CA), and the packaged material was plated on XL1-blue host cells. The library was determined to consist of 2 x 10⁷ V_{H} homologs with less than 30% non-recombinant background.

The vector used above, Lambda Zap II is a derivative of the original Lambda Zap (ATCC # 40,298) that maintains all of the characteristics of the original Lambda Zap including 6 unique cloning sites, fusion protein expression, and the ability to rapidly excise the insert in the form of a phagemid (Bluescript SK-), but lacks the SAM 100 mutation, allowing growth on many Non-Sup F strains, including XL1-Blue. The Lambda Zap II was constructed as described in Short et al., Nucleic Acids Res., 16:7583-7600, 1988, by replacing the Lambda S gene contained in a 4254 base pair (bp) DNA fragment produced by digesting Lambda Zap with the restriction enzyme NcoI. This 4254 bp DNA fragment was replaced with the 4254 bp DNA fragment containing the Lambda S gene isolated from Lambda gt10 (ATCC # 40,179) after digesting the vector with the restriction enzyme NcoI. The 4254 bp DNA fragment isolated from lambda gt10 was ligated into the original Lambda Zap vector using T4 DNA ligase and standard protocols for such procedures described in Current Protocols in Molecular Biology, Ausubel et al., eds., John Wiley and Sons, NY, 1987.

In preparation of cloning a library enriched in V_{L} sequences, 2 µg of PCR amplified products (2.5 mg/30 µl of 150 mM NaCl, 8 mM Tris-HCl (pH 7.5), 6 mM Mg SO₄, 1 mM DTT, 200 mg/ml BSA. 37C) were digested with restriction enzymes Nco I (30 units) and Spe I (45 units). The digested PCR amplified products were purified on a 1% agarose gel using standard electro-elution technique described in Molecular Cloning A Laboratory Manual, Maniatis et al., eds., Cold Spring Harbor, NY, (1982). Briefly, after gel electro-elution of the digested PCR amplified product the region of the gel containing the V_{L}-coding DNA fragment of the appropriate size was excised, electro-elution into a dialysis membrane, ethanol precipitated and re-suspended at a final concentration of 10 ng per ml in a solution containing 10 mM Tris-HCl at pH 7.5 and 1 mM EDTA.

An equal molar amount of DNA representing a plurality of different V_{L}-coding DNA homologs was ligated to a pBluescript SK- phagemid vector that had been previously cut with Nco I and Spe I. A portion of the ligation mixture was transformed using the manufacturer's instructions into Epicuian Coli XL1-Blue competent cells (Stratagene Cloning Systems, La Jolla, CA). The transformant library was determined to consist of 1.2 x 10³ colony forming units/ug of V_{L} homologs with less than 3% non-recombinant background.

### 7. Sequencing of Plasmids from the V_{H}-Coding cDNA Library

To analyze the Lambda Zap II phage clones the clones were excised from Lambda Zap into plasmids according to the manufacture's instructions (Stratagene Cloning System, La Jolla, CA). Clones were first screened for DNA inserts by restriction digests with either Pvu II or Bg1 I and clones containing the putative V_{H} insert were sequenced.

### 8. Characterization Of The Cloned V_{H} Repertoire

The amplified products which had been digested with Xho I and EcoR I and cloned into Lambda ZAP, resulted in a cDNA library with 9.0 x 10⁵ pfu's. In order to confirm that the library consisted of a diverse population of V_{H}-coding DNA homologs, the N-terminal 120 bases of 18 clones, selected at random from the library, were excised and sequenced (Figure 5). To determine if the clones were of V_{H} gene origin, the cloned sequences were compared with known V_{H} sequences and V_{L} sequences. The clones exhibited from 80 to 90% homology with sequences of known heavy chain origin and little homology with sequences of light chain origin when compared with the sequences available in Sequences of Proteins of Immunological Interest by Kabat et al., 4th ed., U.S. Dept.of Health and Human Sciences, (1987). This demonstrated that the library was enriched for the desired V_{H} sequence in preference to other sequences, such as light chain sequences.

The diversity of the population was assessed by classifying the sequenced clones into predefined subgroups (Figure 5). subgroups [I (A,B,), II (A,B,C), III (A,B,C,D,) V (A, B)] based on framework amino acid sequences described in Sequences of Proteins of Immunological Interest by Kabot et al., 4th ed., U.S. Dept.of Health and Human Sciences, (1987); Dildrop, Immunology Today, 5:84, (1984); and Brodeur et al., Eur. J. Immunol., 14; 922, (1984). Classification of the sequenced clones demonstrated that the cDNA library contained V_{H} sequences of at least 7 different subgroups. Further, a pairwise comparison of the homology between the sequenced clones showed that no two sequences were identical at all positions, suggesting that the population is diverse to the extent that it is possible to characterize by sequence analysis.

Six of the clones (L 36-50, Figure 5) belong to the subclass III B and had very similar nucleotide sequences. This may reflect a preponderance of mRNA derived from one or several related variable genes in stimulated spleen, but the data does not permit ruling out the possibility of a bias in the amplification process.

### 9. V_{H}-Expression Vector Construction

To express the plurality of V_{H}-coding DNA homologs in an E. coli host cell, a lambda vector was constructed that placed the V_{H}-coding DNA homologs in the proper reading frame, provided a ribosome binding site as described by Shine et al., Nature, 254:34, 1975, provided a leader sequence directing the expressed protein to the periplasmic space, provided a polynucleotide sequence that coded for a known epitope (epitope tag) and also provided a polynucleotide that coded for a spacer protein between the V_{H}-coding DNA homolog and the polynucleotide coding for the epitope tag. A synthetic DNA sequence containing all of the above polynucleotides and features was constructed by designing single stranded polynucleotide segments of 20-40 bases that would hybridize to each other and form the double stranded synthetic DNA sequence shown in Figure 6. The individual single-stranded polynucleotides (N₁-N₁₂) are shown in Table 3.

The completed synthetic DNA insert was ligated directly into a lambda Zap II vector that had been previously digested with the restriction enzymes Not I and Xho I. The ligation mixture was packaged according to the manufacture's instructions using Gigapack II Gold packing extract available from Stratagene Cloning Systems, La Jolla, CA. The packaged ligation mixture was plated on XL1 blue cells (Stratagene Cloning Systems, San Diego, CA). Individual Lambda Zap II plaques were cored and the inserts excised according to the in vivo excision protocol provided by the manufacturer, Stratagene Cloning Systems, La Jolla, CA. This in vivo excision protocol moves the cloned insert from the Lambda Zap II vector into a plasmid vector to allow easy manipulation and sequencing. The accuracy of the above cloning steps was confirmed by sequencing the insert.

The sequence of the resulting V_{H} expression vector is shown in Figure 6A and Figure 7.

**Table 3**

| | |
|---|---|
| N1) | 5' GGCCGCAAATTCTATTTCAAGGAGACAGTCAT 3' |
| N2) | 5' AATGAAATACCTATTGCCTACGGCAGCCGCTGGATT 3' |
| N3) | 5' GTTATTACTCGCTGCCCAACCAGCCATGGCCC 3' |
| N4) | 5' AGGTGAAACTGCTCGAGAATTCTAGACTAGGTTAATAG 3' |
| N5) | 5' TCGACTATTAACTAGTCTAGAATTCTCGAG 3' |
| N6) | 5' CAGTTTCACCTGGGCCATGGCTGGTTGGG 3' |
| N7) | 5' CAGCGAGTAATAACAATCCAGCGGCTGCCGTAGGCAATAG 3' |
| N8) | 5' GTATTTCATTATGACTGTCTCCTTGAAATAGAATTTGC 3' |
| N9-4) | 5' AGGTGAAACTGCTCGAGATTTCTAGACTAGTTACCCGTAC 3' |
| N11) | 5' GACGTTCCGGACTACGGTTCTTAATAGAATTCG 3' |
| N12) | 5' TCGACGAATTCTATTAAGAACCGTAGTC 3' |
| N10-5) | 5' CGGAACGTCGTACGGGTAACTAGTCTAGAAATCTCGAG 3' |

### 10. V_{L} Expression Vector Construction

To express the plurality of V_{L} coding polynucleotides in an E. coli host cell, a lambda vector was constructed that placed the V_{L} coding polynucleotide in the proper reading frame, provided a ribosome binding site as described by Shine et al., Nature, 254:34, (1975), provided a leader sequence directing the expressed protein to the periplasmic space and also provided a polynucleotide that coded for a spacer protein between the V_{L} polynucleotide and the polynucleotide coding for the epitope tag. A synthetic DNA sequence containing all of the above polynucleotides and features was constructed by designing single stranded polynucleotide segments of 20-40 bases that would hybridize to each other and form the double stranded synthetic DNA sequence shown in Figure 6B. The individual single-stranded polynucleotides (N₁-N₈) are shown in Table 3.

The completed synthetic DNA insert was ligated directly into a Lambda Zap II vector that had been previously digested with the restriction enzymes Not I and Xho I. The sequence of the resulting V_{L} expression vector is shown in Figure 6 and Figure 8.

The V_{L} expression vector used to construct the V_{L} library was the phagemid produced to allow the DNA of the V_{L} expression vector to be determined. The phagemid was produced by the in vivo excision process from the Lambda Zap V_{L} expression vector (Figure 8). The phagemid version of this vector was used because the Nco I restriction enzyme site is unique in this version and thus could be used to operatively linked the V_{L} DNA homologs into the expression vector.

### 11. V_{L}II-Expression Vector Construction

To express the plurality of V_{L}-coding DNA homologs in an E. coli host cell, a vector was constructed that placed the V_{L}-coding DNA homologs in the proper reading frame, provided a ribosome binding site as described by Shine et al., Nature, 254:34, 1975, provided the Pel B gene leader sequence that has been previously used to successfully secrete Fab fragments in E. coli by Lei et al., J. Bac., 169:4379 (1987) and Better et al., Science, 240:1041 (1988), and also provided a polynucleotide containing a restriction endonuclease site for cloning. A synthetic DNA sequence containing all of the above polynucleotides and features was constructed by designing single stranded polynucleotide segments of 20-60 bases that would hybridize to each other and form the double stranded synthetic DNA sequence shown in Figure 10. The sequence of each individual single-stranded polynucleotides (O1-O8) within the double stranded synthetic DNA sequence is shown in Table 4.

The completed synthetic DNA insert was ligated directly into a lambda Zap II vector that had been previously digested with the restriction enzymes Not I and Xho I.

The sequence of the resulting V_{L}II-expression vector is shown in Figure 9 and Figure 11.

**TABLE 4**

| | |
|---|---|
| 01) | 5' 34TGAATTCTAAACTAGTCGCCAAGGAGACAGTCAT 3' |
| 02) | 5' AATGAAATACCTATTGCCTACGGCAGCCGCTGGATT 3' |
| 03) | 5' GTTATTACTCGCTGCCCAACCAGCCATGGCC 3' |
| 04) | 5' GAGCTCGTCAGTTCTAGAGTTAAGCGGCCG 3' |
| 05) | 5' GTATTTCATTATGACTGTCTCCTTGGCGACTAGTTTAGAATTCAAGCT 3' |
| 06) | 5' CAGCGAGTAATAACAATCCAGCGGCTGCCGTAGGCAATAG 3' |
| 07) | 5' TGACGAGCTCGGCCATGGCTGGTTGGG 3' |
| 08) | 5' TCGACGGCCGCTTAACTCTAGAAC 3' |

### 12. V_{H} + V_{L} Library Construction

To prepare an expression library enriched in V_{H} sequences, DNA homologs enriched in V_{H} sequences were prepared according to Example 6 using the same set of 5' primers but with primer 12A (Table 1) as the 3' primer. These homologs were then digested with the restriction enzymes Xho I and Spe I and purified on a 1% agarose gel using the standard electro-elution technique described in Molecular Cloning A Laboratory Manual, Maniatis et al., eds., Cold Spring Harbor, NY, (1982). These prepared V_{H} DNA homologs were then directly inserted into the V_{H} expression vector that had been previously digested with Xho I and Spe I.

The ligation mixture containing the V_{H} DNA homologs were packaged according to the manufacturers specifications using Gigapack Gold II Packing Extract (Stratagene Cloning Systems, La Jolla, CA). The expression libraries were then ready to be plated on XL-1 Blue cells.

To prepare a library enriched in V_{L} sequences, PCR amplified products enriched in V_{L} sequences were prepared according to Example 6. These V_{L} DNA homologs were digested with restriction enzymes Nco I and Spe I. The digested V_{L} DNA homologs were purified on a 1% agarose gel using standard electro-elusion techniques described in Molecular Cloning A Laboratory Manual, Maniatis et al., eds., Cold Spring Harbor, NY (1982). The prepared V_{L} DNA homologs were directly inserted into the V_{L} expression vector that had been previously digested with the restriction enzymes Nco I and Spe I. The ligation mixture containing the V_{L} DNA homologs were transformed into XL-1 blue competent cells using the manufacturer's instructions (Stratagene Cloning Systems, La Jolla, CA).

### 13. Inserting V_{L} Coding DNA Homologs Into V_{L} Expression Vector

In preparation for cloning a library enriched in V_{L} sequences, PCR amplified products (2.5 µg/30 µl of 150 mM NaCl, 8 mM Tris-HCl (pH 7.5), 6 mM MgSO₄, 1 mM DTT, 200 ug/ml BSA at 37°C were digested with restriction enzymes Sac I (125 units) and Xba I (125 units) and purified on a 1% agarose gel. In cloning experiments which required a mixture of the products of the amplification reactions, equal volumes (50 µl, 1-10 ug concentration) of each reaction mixture were combined after amplification but before restriction digestion. After gel electrophoresis of the digested PCR amplified spleen mRNA, the region of the gel containing DNA fragments of approximate 350 bps was excised, electro-eluted into a dialysis membrane, ethanol precipitated and re-suspended in a TE solution containing 10 mM Tris-HC1 pH 7.5 and 1 mM EDTA to a final concentration of 50 ng/µl.

The V_{L}II-expression DNA vector was prepared for cloning by admixing 100 µg of this DNA to a solution containing 250 units each of the restriction endonucleases Sac 1 and Xba 1 (both from Boehringer Mannheim, Indianapolis, IN) and a buffer recommended by the manufacturer. This solution was maintained at 37°C from 1.5 hours. The solution was heated at 65°C for 15 minutes top inactivate the restriction endonucleases. The solution was chilled to 30°C and 25 units of heat-killable (HK) phosphatase (Epicenter, Madison, WI) and CaCl₂ were admixed to it according to the manufacturer's specifications. This solution was maintained at 30C for 1 hour. The DNA was purified by extracting the solution with a mixture of phenol and chloroform followed by ethanol precipitation. The V_{L}II expression vector was now ready for ligation to the V_{L} DNA homologs prepared in the above examples.

DNA homologs enriched in V_{L} sequences were prepared according to Example 5 but using a 5' light chain primer and the 3' light chain primer shown in Table 2. Individual amplification reactions were carried out using each 5' light chain primer in combination with the 3' light chain primer. These separate V_{L} homolog containing reaction mixtures were mixed and digested with the restriction endonucleases Sac 1 and Xba 1 according to Example 6. The V_{L} homologs were purified on a 1% agarose gel using the standard electro-elution technique described in Molecular Cloning A Laboratory Manual, Maniatis et al., eds., Cold Spring Harbor, NY, (1982). These prepared V_{L} DNA homologs were then directly inserted into the Sac 1 - Xba cleaved V_{L}II-expression vector that was prepared above by ligating 3 moles of V_{L} DNA homolog inserts with each mole of the V_{L}II-expression vector overnight at 5C. 3.0 x 10⁵ plague forming units were obtained after packaging the DNA with Gigapack II Bold (Stratagene Cloning Systems, La Jolla, CA) and 50% were recombinants.

### 14. Randomly Combining V_{H} and V_{L} DNA Homologs on the Same Expression Vector

The V_{L}II-expression library prepared in Example 13 was amplified and 500 µg of V_{L}II-expression library phage DNA prepared from the amplified phage stock using the procedures described in Molecular Cloning: A Laboratory Manual, Maniatis et al., eds., Cold Spring Harbor, NY (1982), 50 µg of this V_{L}II-expression library phage DNA was maintained in a solution containing 100 units of MLuI restriction endonuclease (Boehringer Mannheim, Indianapolis, IN) in 200 µl of a buffer supplied by the endonuclease manufacturer for 1.5 hours at 37°C. The solution was then extracted with a mixture of phenol and chloroform. The DNA was then ethanol precipitated and re-suspended in 100 µl of water. This solution was admixed with 100 units of the restriction endonuclease EcoR I (Boehringer Mannheim, Indianapolis, IN) in a final volume of 200 µl of buffer containing the components specified by the manufacturer. This solution was maintained at 37°C for 1.5 hours and the solution was then extracted with a mixture of phenol and chloroform. The DNA was ethanol precipitated and the DNA re-suspended in TE.

The V_{H} expression library prepared in Example 12 was amplified and 500 ug of V_{H} expression library phage DNA prepared using the methods detailed above. 50 µg of the V_{H} expression library phage DNA was maintained in a solution containing 100 units of Hind III restriction endonuclease (Boehringer Mannheim, Indianapolis, IN) in 200 µl of a buffer supplied by the endonuclease manufacturer for 1.5 hours at 37°C. The solution was then extracted with a mixture of phenol and chloroform saturated with 0.1 M Tris-HCl at pH 7.5. The DNA was then ethanol precipitated and re-suspended in 100 µl of water. This solution was admixed with 100 units of the restriction endonuclease EcoR I (Boehringer Mannheim, Indianapolis, IN) in a final volume of 200 µl of buffer containing the components specified by the manufacturer. This solution was maintained at 37°C for 1.5 hours and the solution was then extracted with a mixture of phenol and chloroform. The DNA was ethanol precipitated and the DNA re-suspended in TE.

The restriction digested V_{H} and V_{L}II-expression Libraries were ligated together. The ligation reaction consisted of 1 µg of V_{H} and 1 µg of V_{L}II phage library DNA in a 10 µl reaction using the reagents supplied in a ligation kit purchased from Stratagene Cloning Systems (La Jolla, California). After ligation for 16 hr at 4°C, 1 µl of the ligated the phage DNA was packaged with Gigapack Gold II packaging extract and plated on XL 1-blue cells prepared according to the manufacturers instructions. A portion of the 3X10⁶ clones obtained were used to determine the effectiveness of the combination. The resulting V_{H} and V_{L} expression vector is shown in Figure 11.

Clones containing both V_{H} and V_{L} were excised from the phage to pBluescript using the in vitro excision protocol described by Short et al., Nucleic Acid Research, 16:7583-7600 (1988). Clones chosen for excision expressed the decapeptide tag and did not cleave X-gal in the presence of 2mM IPTGthus remaining white. Clones with these characteristics represented 30% of the library. 50% of the clones chosen for excision contained a v_{H} and V_{L} as determined by restriction analysis. Since approximately 30% of the clones in the V_{H} library expressed the decapeptide tag and 50% of the clones in the V_{L}II library contained a V_{L} sequence it was anticipated that no more than 15% of the clones in the combined library would contain both V_{H} and V_{L} clones. The actual number obtained was 15% of the library indicating that the process of combination was very efficient.

### 15. Segregating DNA Homologs For a V_{H} Antigen Binding Protein

To segregate the individual clones containing DNA homologs that code for a V_{H} antigen binding protein, the titre of the V_{H} expression library prepared according to Example 11 was determined. This library titration was performed using methods well known to one skilled in the art.

The titred expression library was then plated out so that replica filters could be made from the library. The replica filters will be used to later segregate out the individual clones in the library that are expressing the antigens binding proteins of interest. Briefly, a volume of the titred library that would yield 20,000 plaques per 150 millimeter plate was added to 600 µl of exponentially growing E. coli cells and maintained at 37°C for 15 minutes to allow the phage to absorb to the bacterial cells. Then 7.5 ml of top agar was admixed to the solution containing the bacterial cells and the absorbed phage and the entire mixture distributed evenly across the surface of a prewarmed bacterial agar plate. This process was repeated for a sufficient number of plates to plate out a total number of plaques at least equal to the library size. These plates were then maintained at 37°C for 5 hours. The plates were then overlaid with nitrocellulose filters that had been pretreated with a solution containing 10 mM isopropylbeta-D-thiogalactopyranosid (IPTG) and maintained at 37°C for 4 hours. The orientation of the nitrocellulose filters in relation to the plate were marked by punching a hole with a needle dipped in waterproof ink through the filter and into the bacterial plates at several locations. The nitrocellulose filters were removed with forceps and washed once in a TBST solution containing 20 mM Tris-HCL at pH 7.5, 150 mM NaCl and 0.05% monolaurate (tween-20). A second nitrocellulose filter that had also been soaked in a solution containing 10 mM IPTG was reapplied to the bacterial plates to produce duplicate filters. The filters were further washed in a fresh solution of TBST for 15 minutes. Filters were then placed in a blocking solution consisting of 20 mM Tris-HCl at pH 7.5, 150 mM NaCL and 1% BSA and agitated for 1 hour at room temperature. The nitrocellulose filters were transferred to a fresh blocking solution containing a 1 to 500 dilution of the primary antibody and gently agitated for at least 1 hour at room temperature. After the filters were agitated in the solution containing the primary antibody the filters were washed 3 to 5 times in TBST for 5 minutes each time to remove any of the residual unbound primary antibody. The filters were transferred into a solution containing fresh blocking solution and a 1 to 500 to a 1 to 1,000 dilution of alkaline phosphatase conjugated secondary antibody. The filters were gently agitated in the solution for at least 1 hour at room temperature. The filters were washed 3 to 5 times in a solution of TBST for at least 5 minutes each time to remove any residual unbound secondary antibody. The filters were washed once in a solution containing 20 mM Tris-HCl at pH 7.5 and 150 mM NaCL. The filters were removed from this solution and the excess moisture blotted from them with filter paper. The color was developed by placing the filter in a solution containing 100 mM Tris-HCl at pH 9.5, 100 mM NaCl, 5 mM MgCl₂, 0.3 mg/ml of nitro Blue Tetrazolium (NBT) and 0.15 mg/ml of 5-bromo-4-chloro-3-indolyl-phosphate (BCIP) for at least 30 minutes at room temperature. The residual color development solution was rinsed from the filter with a solution containing 20 mM Tris-HCl at pH 7.5 and 150 mM NaCl. The filter was then placed in a stop solution consisting of 20 mM Tris-HCl at pH 2.9 and 1 mM EDTA. The development of an intense purple color indicates at positive result. The filters are used to locate the phage plaque that produced the desired protein. That phage plaque is segregated and then grown up for further analysis.

Several different combinations of primary antibodies and second antibodies were used. The first combination used a primary antibody immunospecific for a decapeptide that will be expressed only if the V_{H} antigen binding protein is expressed in the proper reading frame to allow read through translation to include the decapeptide epitope covalently attached to the V_{H} antigen binding protein. This decapeptide epitope and an antibody immunospecific for this decapeptide epitope was described by Green et al., Cell 28:477 (1982) and Niemann et al., Proc. Nat. Acad. Sci. U.S.A. 80:4949 (1983). The sequence of the decapeptide recognized is shown in Figure 11. A functional equivalent of the monoclonal antibody that is immunospecific for the decapeptide can be prepared according to the methods of Green et al. and Niemann et al. The secondary antibody used with this primary antibody was a goat anti-mouse IgG (Fisher Scientific). This antibody was immunospecific for the constant region of mouse IgG and did not recognize any portion of the variable region of heavy chain. This particular combination of primary and secondary antibodies when used according to the above protocol determined that between 25% and 30% of the clones were expressing the decapeptide and therefore these clones were assumed to also be expressing a V_{H} antigen binding protein.

In another combination the anti-decapeptide mouse monoclonal was used as the primary antibody and an affinity purified goat anti-mouse Ig, commercially available as part of the picoBlue immunoscreening kit from Stratagene Cloning System, La Jolla, CA, was use as the secondary antibody. This combination resulted in a large number of false positive clones because the secondary antibody also immunoreacted with the V_{H} of the heavy chain Therefore this antibody reacted with all clones expressing any V_{H} protein and this combination of primary and secondary antibodies did not specifically detect clones with the V_{H} polynucleotide in the proper reading frame and thus allowing expressing of the decapeptide.

Several combinations of primary and secondary antibodies are used where the primary antibody is conjugated to fluorescein isothiocyanate (FITC) and thus the immunospecificity of the antibody was not important because the antibody is conjugated to the preselected antigen (FITC) and it is that antigen that should be bound by the V_{H} antigen binding proteins produced by the clones in the expression library. After this primary antibody has bound by virtue that is FITC conjugated mouse monoclonal antibody p2 5764 (ATCC #HB-9505). The secondary antibody used with this primary antibody is a goat anti-mouse Ig⁶ (Fisher Scientific, Pittsburg, PA) conjugated to alkaline phosphatase. Using the method described in Antibodies A Laboratory Manual, Harlow and Lowe, eds., Cold Springing Harbor, NY, (1988). If a particular clone in the V_{H} expression, library, expresses a V_{H} binding protein that binds the FITC covalently coupled to the primary antibody, the secondary antibody binds specifically and when developed the alkaline phosphate causes a distinct purple color to form.

The second combination of antibodies of the type uses a primary antibody that is FITC conjugated rabbit anti-human IgG (Fisher Scientific, Pittsburg, PA). The secondary antibody used with this primary antibody is a goat anti-rabbit IgG conjugated to alkaline phosphatase using the methods described in Antibodies A Laboratory Manual, Harlow and Lane, eds., Cold Spring Harbor, NY, (1988). If a particular clone in the V_{H} expression library expresses a V_{H} binding protein that binds the FITC conjugated to the primary antibody, the secondary antibody binds specifically and when developed the alkaline phosphatase causes a distinct purple color to form.

Another primary antibody was the mouse monoclonal antibody p2 5764 (ATCC # HB-9505) conjugated to both FITC and ¹²⁵I. The antibody would be bound by any V_{H} antigen binding proteins expressed. Then because the antibody is also labeled with ¹²⁵I, an autoradiogram of the filter is made instead of using a secondary antibody that is conjugated to alkaline phosphatase. This direct production of an autoradiogram allows segregation of the clones in the library expressing a V_{H} antigen binding protein of interest.

### 16. Segregating DNA Homologs For a V_{H} and V_{L} that Form an Antigen Binding F_{V}

To segregate the individual clones containing DNA homologs that code for a V_{H} and a V_{L} that form an antigen binding F_{V} the V_{H} and V_{L} expression library was titred according to Example 15. The titred expression library was then screened for the presence of the decapeptide tag expressed with the V_{H} using the methods described in Example 15. DNA was then prepared from the clones to express the decapeptide tag. This DNA was digested with the restriction endonuclease Pvu II to determine whether these clones also contained a V_{L} DNA homolog. The slower migration of a PvuII restriction endonuclease fragment indicated that the particular clone contained both a V_{H} and a V_{L} DNA homolog.

The clones containing both a V_{H} and a V_{L} DNA homolog were analyzed to determine whether these clones produced an assembled F_{V} protein molecule from the V_{H} and V_{L} DNA homologs.

The F_{V} protein fragment produced in clones containing both V_{H} and V_{L} was visualized by immune precipitation of radiolabeled protein expressed in the clones, using anti-decapeptide monoclonal antibody and protein G coupled to sepharose beads.

The resulting autoradiogram is shown in Figure 12. The presence of assembled F_{V} molecules can be seen by the presence of V_{L} that was immunoprecipitated because it was attached to the V_{H}-decapeptide tag recognized by the precipitating antibody.

### 17. Construction of Selectable V_{H} and V_{L} Expression

### A. Construction of the Mutant S Gene Expression Plasmid

The bacteria phage lambda S gene has been shown to be directly involved in lysis as described by Reader et al., Virology, 43:607-622 (1971). The S gene encodes a 107 amino acid polypeptide that is responsible for a lethal event in the cytoplasmic membrane that allows the release of the R gene product into the periplasm of the cell where it degrades the peptidoglycan as described by Garrett et al., J. Virology, 44:886-892 (1982). The dominant S gene mutant (S₁₀₀ SAM 5) is a mutation that has been shown to interfere with the formation of the normal S protein membrane channel thus preventing cell lysis. See Raab et al., J. Mol. Biol., 199:95-105 (1988). This mutant S gene is dominant because when it is expressed, even in the presence of the wild type S protein it prevents lysis of the bacterial cell. The S₁₀₀ SAM 5 dominant mutation also contains an amber mutation and therefore requires the expression of a suppressor tRNA in the bacterial cell in order for mutant S protein to be produced. Further, this amber mutation allows the growth of bacteria containing the mutant S gene construct without lysis because without this amber suppressing tRNA no functional S gene protein is produced.

The dominant S gene from Lambda Zap Sam 5 was isolated using the polymerase chain reaction. Briefly, Lambda Zap Sam 5 DNA was isolated using the methods described in Molecular Cloning: A Laboratory Manual, Maniatis et al., eds., Cold Spring Harbor, NY (1982).

The mutant S gene DNA produced by the above polymerase chain reaction was digested with the restriction endonucleases Hind III and Bgl II.

The mutant S gene was inserted into pBluescript KS+ (Stratagene) that had been previously digested with the restriction endonuclease Hind III and BamH I.

The pBluescript KS+ vector with the insert was transformed in XL1 Blue cells (Stratagene) according to the manufacture's directions.

The accuracy of the above cloning steps is confirmed by DNA sequencing.

### B. Selectable V_{H}-Expression Vector Construction

To add the ability to select against expression vectors not containing v_{H}-coding DNA homologs, a suppressor tRNA gene was inserted into the V_{H}-Expression vector prepared in Example 9. The selectable V_{H}-Expression vector was prepared by inserting a synthetic DNA sequence containing the suppressor tRNA gene and DNA sequence coding for the decapeptide tag into the V_{H}-Expression vector prepared in Example 9 that had been previously cleaved with the restriction endonucleases Xho I and Eco RI.

A synthetic DNA sequence containing the suppressor tRNA gene and polynucleotide sequence coding for decapeptide tag was constructed by designing single stranded polynucleotide segments of 20-40 bases that would hybridize to each other and form the double stranded synthetic DNA sequence shown in Figure 18A. The individual single-stranded polynucleotides are shown in Table 5.

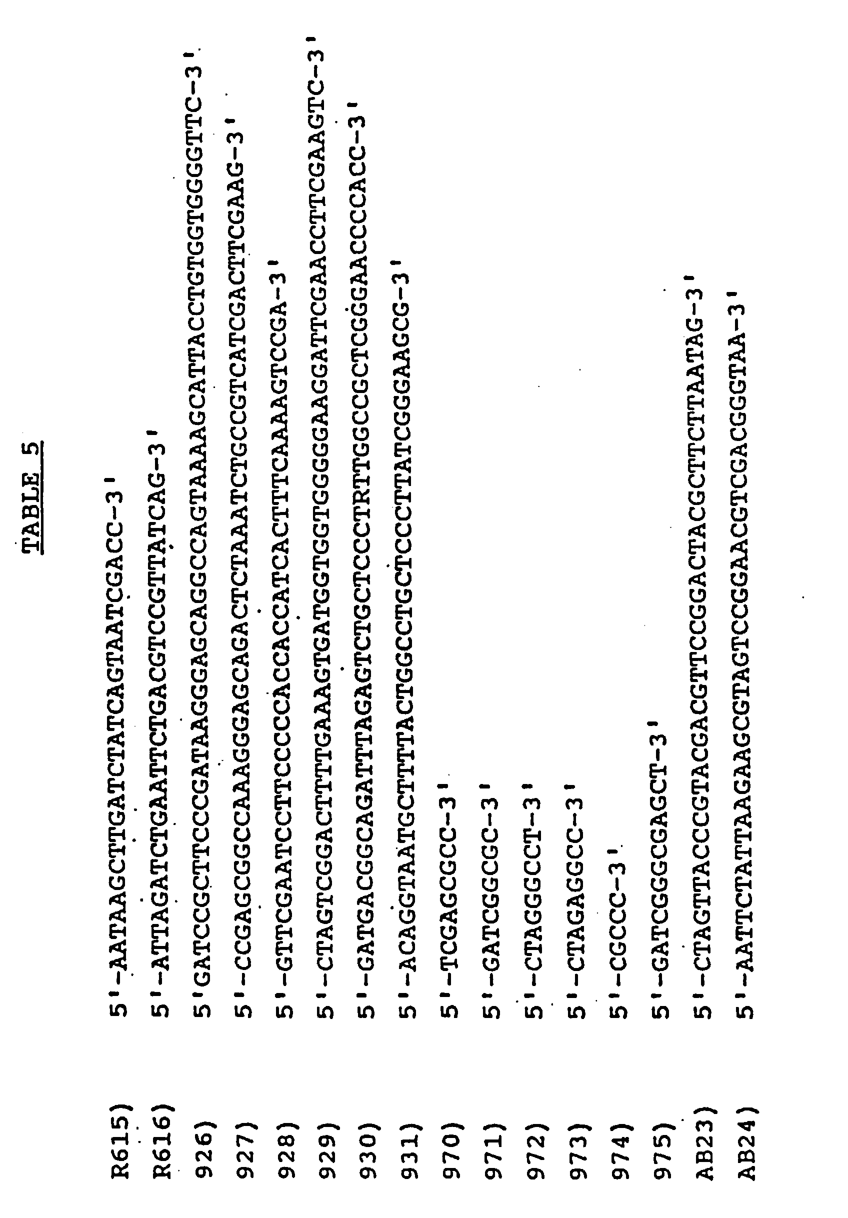

The required polynucleotides were annealed to form the synthetic DNA sequence shown in Figure 18A.

The completed synthetic DNA insert (Figure 18A) was ready for ligation to the V_{H}-Expression vector (Figure 7) that had been previously digested with the restriction endonucleases Xho I and Eco RI.

The ligation mixture was then packaged according to the manufacturer's instructions using Gigapack II Gold packing extract available from Stratagene. The packaged ligation mixture was then plated on XL1 blue cells (Stratagene).

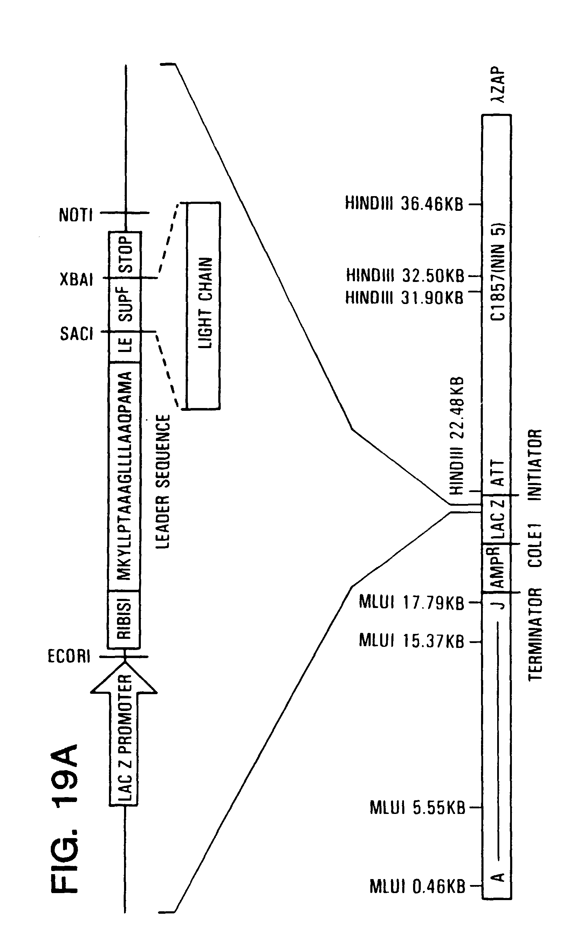

Individual lambda phage plaques were selected for DNA sequencing by selecting individual plaques that hybridized to polynucleotides contained in the synthetic DNA insert according to the method described in Current Protocols in Molecular Biology, Ausubel et al., eds., John Wiley and Sons, NY (1987). The selectable V_{H} expression vector is shown in Figure 19A.

### C. Selectable V_{L}-Expression Vector Construction

To add the ability to select against expression vectors not containing V_{L}-coding DNA homologs, a suppressor tRNA gene was inserted into the V_{L}-Expression vector prepared in Example 11. The selectable V_{L}-Expression vector was prepared by inserting a synthetic DNA sequence containing the suppressor tRNA gene into the V_{L}-Expression vector prepared in Example 11 that had been previously cleaved with the restriction endonucleases Sac I and Xba I.

A synthetic DNA sequence containing the Suppressor tRNA gene was constructed by designing single stranded polynucleotide segments of 20-40 bases that would hybridize to each other and form the double stranded synthetic DNA sequence shown in Figure 18B. The individual single-stranded polynucleotides are shown in Table 5.

The required polynucleotides were annealed to form the synthetic DNA sequence shown in Figure 18B.

The completed synthetic DNA insert (Figure 18B) was ready for ligation to the V_{L}-Expression vector (Figure 9) that had been previously digested with the restriction endonucleases Sac I and Xba I.

The synthetic DNA insert prepared above was inserted by ligation into the restriction endonuclease cleaved V_{L}-Expression vector.

The ligation mixture was packaged according to the manufacturer's instructions using Gigapack II Gold packing extract available from Stratagene. The packaged ligation mixture was plated on XL1 blue cells (Stratagene).

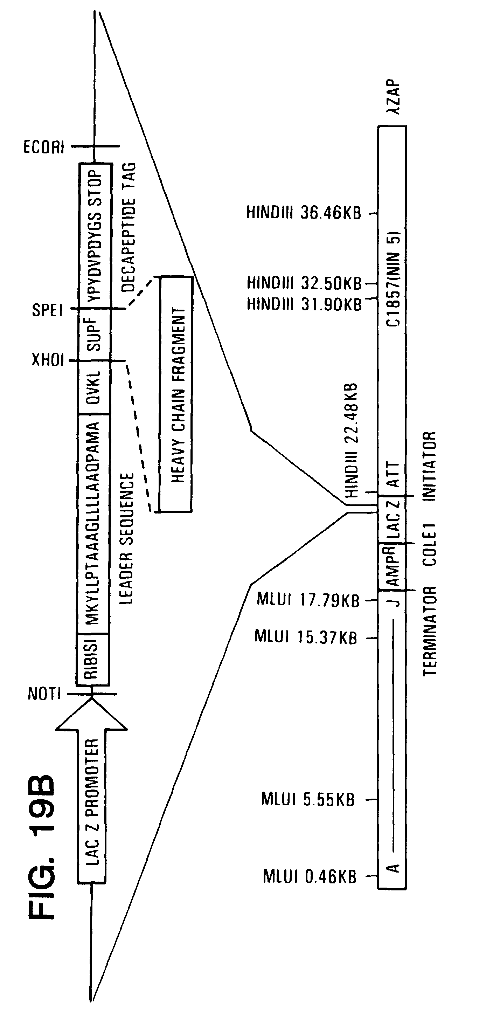

Individual lambda phage plaques were selected for DNA sequencing by screening for plaques that hybridized to polynucleotides contained in the synthetic DNA insert according to the methods described in Molecular Cloning: A Laboratory Manual, Maniatis et al., eds., Cold Spring Harbor, NY (1989). The selectable V_{L} expression vector is shown in Figure 19B.

### D. Construction of a Selectable V_{L} and V_{H}- Expression Vector

The V_{H}-Expression vector prepared in Example 17B is modified so that it does not contain any Xho I or Spe I restriction endonuclease sites. This modification of this vector is accomplished using a set of polynucleotides and methods similar to the methods described in Example 17B.

The V_{L}-Expression vector prepared in Example 17C is modified so that it does not contain any Sac I or Xba I restriction endonuclease sites. This modification of the V_{L}-Expression vector is accomplished using a set of polynucleotides and methods well known in the art and similar to the methods described in Example 17C. The modified V_{L}-Expression vector and the modified V_{H}-Expression vector are combined to produce a selectable V_{L} and V_{H} Expression vector. Briefly, the modified V_{H}-Expression vector is digested with the restriction endonucleases Eco RI and Hind III using the conditions recommended by the enzyme manufacturer and is digested with the restriction endonucleases Eco RI and Mlu I. The restriction endonuclease cleaved V_{H} and V_{L} Expression vectors are the ligated together using standard techniques to form the selectable V_{H} and V_{L} Expression vector shown in Figure 20.

The V_{H} and V_{L} Expression vector contains 2 suppressor tRNA genes, one is replaced by the V_{H} DNA homolog and the other is replaced by the V_{L} DNA homolog. Therefore, when the vector contains both a V_{H} and a V_{L} DNA homolog, the vector does not contain a suppressor tRNA gene allowing the V_{H} and V_{L} containing vector to produce phage plaques under the appropriate selection conditions.

### E. Inserting DNA Homologs into the Selectable DNA Expression Vectors

V_{H} coding and/or V_{L} coding DNA homologs prepared in Example 5 are inserted into the V_{H} and V_{L} expression vector, the V_{H} expression vector, or the V_{L} expression vector using the provided restriction endonuclease sites. The V_{H} coding DNA homologs are typically inserted into the provided Xho I and Spe I restriction endonuclease sites (Figure 20) using standard procedures. The V_{L} coding DNA homologs are typically inserted into the provided restriction endonuclease sites (Figure 20). Therefore, depending on the particular expression vector selected, the methods described herein produce an expression vector containing a V_{H} coding DNA homolog alone, a V_{L} coding DNA homolog alone, or a V_{H} and a V_{L} DNA homolog.

The V_{H} coding DNA homologs may be inserted into the expression vector first, followed by the V_{L} DNA homologs. Alternatively, the V_{L} coding homologs may be inserted first followed by the V_{H} coding homologs. Either insertion order allows the random recombination of a library of V_{H} coding DNA homologs with a library V_{L} coding DNA homologs. After the V_{H} homologs have been inserted into the V_{H} + V_{L} expression vector, the expression vector can be grown to produce more of the V_{H} containing expression vector. The V_{L} coding DNA homologs can then be inserted into the V_{H} and V_{L} expression vector. Any of these procedures will allow the production of a large combinatorial library.

### F. Selection of V_{H} and/or V_{L} DNA Homolog Containing Phage

A strong selection system is employed in order to reduce the number of expression vectors present in the final library that do not contain V_{H} and/or V_{L} DNA homologs. The selection system combines the dominant Lambda S gene mutation with the suppressor tRNA that is present in V_{H} and/or V_{L} expression vectors. When the suppressor tRNA is present in the expression vector, the mutant Lambda S protein is produced preventing the lysis of the infected cell and thereby preventing the formation of a phage plaque. When a DNA homolog replaces the suppressor tRNA, the expression vector can produce a phage plaque. In order to detect a V_{H} and/or V_{L} the V_{H} and/or V_{L} expression vector must produce a phage plaque because without plaque production there is not enough V_{H} and V_{L} expressed to detect using either immunologic or binding assays. Therefore, phages not containing a V_{H} and/or V_{L} will not be detected. To accomplish this selection, appropriate host bacterial cells containing the mutant S gene plasmid produced in Example 17A are infected with the desired expression vector library. Only the expression vectors without suppressor tRNA genes, the expression vectors containing DNA homologs, produce phage plaques.

### 18. Generation of a Large Combinatorial Library of the Immunoglobulin Repertoire in Phage

Vectors suitable for expression of V_{H}, V_{L}, Fv and Fab sequences are diagrammed in Figures 7 and 9. As previously discussed, the vectors were constructed by modification of Lambda Zap by inserting synthetic oligonucleotides into the multiple cloning site. The vectors were designed to be antisymmetric with respect to the Not I and EcoR I restriction sites which flank the cloning and expression sequences. As described below, this antisymmetry in the placement of restriction sites in a linear vector like bacteriophage is the essential feature of the system which allows a library expressing light chains to be combined with one expressing heavy chains to construct combinatorial Fab expression libraries. Lambda Zap II V_{L}II (Figure 9) is designed to serve as a cloning vector for light chain fragments and Lambda Zap II V_{H} (Figure 7) is designed to serve as a cloning vector for heavy chain sequences in the initial step of library construction. These vectors are engineered to efficiently clone the products of PCR amplification with specific restriction sites incorporated at each end.

### A. PCR Amplification of Antibody Fragments

The PCR amplification of mRNA isolated from spleen cells with oligonucleotides which incorporate restriction sites into the ends of the amplified product can be used to clone and express heavy chain sequences including Fd and kappa chain sequences. The oligonucleotide primers used for these amplifications are presented in Tables 1 and 2. The primers are analogous to those which have been successfully used in Example 5 for amplification of V_{H} sequences. The set of 5' primers for heavy chain amplification were identical to those previously used to amplify V_{H} and those for light chain amplification were chosen on similar principles, Sastry et al., Proc. Natl. Acad. Sci. USA, 8G: 5728 (1989) and Orlandi et al., Proc. Natl. Acad. Sci. USA, 8G:3833 (1989). The unique 3' primers of heavy (IgG1) and light (k) chain sequences were chosen to include the cysteines involved in heavy-light chain disulfide bond formation. At this stage no primer was constructed to amplify lambda light chains since they constitute only a small fraction of murine antibodies. In addition, Fv fragments have been constructed using a 3' primer which is complementary to the mRNA in the J (joining) region (amino acid 128) and a set of unique 5' primers which are complementary to the first strand cDNA in the conserved N-terminal region of the processed protein. Restriction endonuclease recognition sequences are incorporated into the primers to allow for the cloning of the amplified fragment into a lambda phage vector in a predetermined reading frame for expression.

### B. Library Construction

The construction of a combinatorial library was accomplished in two steps. In the first step, separate heavy and light chain libraries were constructed in Lambda Zap II V_{H} and Lambda Zap II V_{L} II respectively. In the second step, these two libraries were combined at the antisymmetric EcoR1 sites present in each vector. This resulted in a library of clones each of which potentially co-expresses a heavy and a light chain. The actual combinations are random and do not necessarily reflect the combinations present in the B-cell population in the parent animal. Lambda Zap II V_{H} expression vector has been used to create a library of heavy chain sequences from DNA obtained by PCR amplification of mRNA isolated from the spleen of a 129 Gᵢₓ + mouse previously immunized with p-nitrophenyl phosphonamidate (NPN) antigen 1 according to formula I (Figure 13) conjugated to keyhole limpet hemocyanin (KLH). The NPN-KLH conjugate was prepared by admixture of 250 µl of a solution containing 2.5 mg of NPN according to formula 1 (Figure 13) in dimethylformamide with 750 µl of a solution containing 2 mg of KLH in 0.01 M sodium phosphate buffer (pH 7.2). The two solutions were admixed by slow addition of the NPN solution to the KLH solution while the KLH solution was being agitated by a rotating stirring bar. Thereafter the admixture was maintained at 4° for 1 hour with the same agitation to allow conjugation to proceed. The conjugated NPN-KLH was isolated from the nonconjugated NPN and KLH by gel filtration through Sephadex G-25. The isolated NPN-KLH conjugate was used in mouse immunizations as described in Example 2.

The spleen mRNA resulting from the above immunizations was isolated and used to create a primary library of V_{H} gene sequences using the Lambda Zap II V_{H} expression vector. The primary library contains 1.3 x 10⁶ pfu and has been screened for the expression of the decapeptide tag to determine the percentage of clones expressing Fd sequences. The sequence for this peptide is only in frame for expression following the cloning of a Fd (or V_{H}) fragment into the vector. At least 80% of the clones in the library express Fd fragments based on immuno-detection of the decapeptide tag.

The light chain library was constructed in the same way as the heavy chain and shown to contain 2.5 x 10⁶ members. Plaque screening, using an anti-kappa chain antibody, indicated that 60% of the library contained expressed light chain inserts. This relatively small percentage of inserts probably resulted from incomplete dephosphorylation of vector after cleavage with Sac I and Xba I.

Once obtained, the two libraries were used to construct a combinatorial library by crossing them at the EcoR I site. To accomplish the cross, DNA was first purified from each library. The light chain library was cleaved with MluI restriction endonuclease, the resulting 5' ends dephosphorylated and the product digested with EcoR I. This process cleaved the left arm of the vector into several pieces but the right arm containing the light chain sequences, remained intact. In a parallel fashion, the DNA of heavy chain library was cleaved with HindIII, dephosphorylated and cleaved with EcoR I, destroying the right arm but leaving the left arm containing the heavy chain sequences intact. The DNA's so prepared were then combined and ligated. After ligation only clones which resulted from combination of a right arm of light chain-containing clones and a left arm of heavy chain-containing clones reconstituted a viable phage. After ligation and packaging, 2.5 x 10⁷ clones were obtained. This is the combinatorial Fab expression library that was screened to identify clones having affinity for NPN. To determine the frequency the phage clones which co-express the light and heavy chain fragments, duplicate lifts of the light chain, heavy chain and combinatorial libraries were screened as above for light and heavy chain expression. In this study of approximately 500 recombinant phage approximately 60% co-expressed light and heavy chain proteins.

### C. Antigen Binding

All three libraries, the light chain, the heavy chain and Fab were screened to determine if they contained recombinant phage that expressed antibody fragments binding NPN. In a typical procedure 30,000 phage were plated and duplicate lifts with nitrocellulose screened for binding to NPN coupled to ¹²⁵I labeled BSA (Figure 15). Duplicate screens of 80,000 recombinant phage from the light chain library and a similar number from the heavy chain library did not identify any clones which bound the antigen. In contrast, the screen of a similar number of clones from the Fab expression library identified many phage plaques that bound NPN (Figure 15). This observation indicates that under conditions where many heavy chains in combination with light chains bind to antigen the same heavy or light chains alone do not. Therefore, in the case of NPN, it is believed that there are many heavy and light chains that only bind antigen when they are combined with specific light and heavy chains respectively.

To assess the ability to screen large numbers of clones and obtain a more quantitative estimate of the frequency of antigen binding clones in the combinatorial library, one million phage plaques were screened and approximately 100 clones which bound to antigen were identified. For six clones which were believed to bind NPN, a region of the plate containing the positive and approximately 20 surrounding bacteriophage plaques was "cored", replated, and screened with duplicate lifts (Figure 15). As expected, approximately one in twenty of the phage specifically bind to antigen. "Cores" of regions of the plated phage believed to be negative did not give positives on replating.

To determine the specificity of the antigen-antibody interaction, antigen binding was competed with free unlabeled antigen as shown in Figure 16. Competition studies showed that individual clones could be distinguished on the basis of antigen affinity. The concentration of free antigen required for complete inhibition of binding varied between 10-100 x 10⁹ M suggesting that the expressed Fab fragments had binding constants in the nanomolar range.

### D. Composition of the Clones and Their Expressed Products

In preparation for characterization of the protein products able to bind NPN as described in Example 18C, a plasmid containing the heavy and light chain genes was excised from the appropriate "cored" bacteriophage plaque using M13mp8 helper phage. Mapping of the excised plasmid demonstrated a restriction pattern consistent with incorporation of heavy and light chain sequences. The protein products of one of the clones was analyzed by ELISA and Western blotting to establish the composition of the NPN binding protein. A bacterial supernate following IPTG induction was concentrated and subjected to gel filtration. Fractions in the molecular weight range 40-60 kD were pooled, concentrated and subjected to a further gel filtration separation. As illustrated in Figure 17, ELISA analysis of the eluting fractions demonstrated that NPN binding was associated with a protein of molecular weight about 50 kD which immunological detection showed contained both heavy and light chains. A Western blot (not shown) of a concentrated bacterial supernate preparation under non-reducing conditions was developed with anti-decapeptide antibody. This revealed a protein band of molecular weight of 50 kD. Taken together these results are consistent with NPN binding being a function of Fab fragments in which heavy and light chains are covalently linked.

### E. Comparison of the Properties of the In Vivo Repertoire Versus the Phage Combinatorial Library

In this example a relatively restricted library was prepared because only a limited number of primers were used for PCR amplification of Fd sequences. The library is expected to contain only clones expressing kappa/gamma1 sequences. However, this is not an inherent limitation of the method since additional primers can be added to amplify any antibody class or subclass. Despite this restriction we were able to isolate a large number of antigen binding clones.

A central issue arising from this work is how a phage library prepared as described herein compares with the in vivo antibody repertoire in terms of size, characteristics of diversity, and ease of access.

The size of the mammalian antibody repertoire is difficult to judge but a figure of the order of 10⁶-10⁸ different antigen specificities is often quoted. With some of the reservations discussed below, a phage library of this size or larger can readily be constructed by a modification of the current method. In fact once an initial combinatorial library has been constructed, heavy and light chains can be shuffled to obtain libraries of exceptionally large numbers.

In principle, the diversity characteristics of the naive (unimmunized) in vivo repertoire and corresponding phage library are expected to be similar in that both involve a random combination of heavy and light chains. However, different factors will act to restrict the diversity expressed by an in vivo repertoire and phage library. For example a physiological modification such as tolerance will restrict the expression of certain antigenic specificities from the in vivo repertoire but these specificities may still appear in the phage library. On the other hand, bias in the cloning process may introduce restrictions into the diversity of the phage library. For example the representation of mRNA for sequences expressed by stimulated B-cells can be expected to predominate over those of unstimulated cells because of higher levels of expression. Different source tissues (e.g., peripheral blood, bone marrow or regional lymph nodes) and different PCR primers (e.g., ones expected to amplify different antibody classes) may result in libraries with different diversity characteristics.

Another difference between in vivo repertoire and phage library is that antibodies isolated from the former may have benefited from affinity maturation due to somatic mutations after combination of heavy and light chains whereas the latter randomly combines the matured heavy and light chains. Given a large enough phage library derived from a particular in vivo repertoire, the original matured heavy and light chains will be recombined. However, since one of the potential benefits of this new technology is to obviate the need for immunization by the generation of a single highly diverse "generic" phage library, it would be useful to have methods to optimize sequences to compensate for the absence of somatic mutation and clonal selection. Three procedures are made readily available through the methods of the present invention. First, saturation mutagenesis may be performed on the CDR's and the resulting Fabs can be assayed for increased function. Second, a heavy or a light chain of a clone which binds antigen can be recombined with the entire light or heavy chain libraries respectively in a procedure identical to the one used to construct the combinatorial library. Third, iterative cycles of the two above procedures can be performed to further optimize the affinity or catalytic properties of the immunoglobulin. It should be noted that the latter two procedures are not permitted in B-cell clonal selection which suggests that the methods described here may actually increase the ability to identify optimal sequences.

Access is the third area where it is of interest to compare the in vivo antibody repertoire and phage library. In practical terms the phage library is much easier to access. The screening methods allow one to survey at least 50,000 clones per plate so that 10⁶ antibodies can be readily examined in a day. This factor alone should encourage the replacement of hybridoma technology with the methods described here. The most powerful screening methods utilize selection which may be accomplished by incorporating selectable markers into the antigen such as leaving groups necessary for replication of auxotrophic bacterial strains or toxic substituents susceptible to catalytic inactivation. There are also further advantages related to the fact that the in vivo antibody repertoire can only be accessed via immunization which is a selection on the basis of binding affinity. The phage library is not similarly restricted. For example, the only general method to identify antibodies with catalytic properties has been by pre-selection on the basis of affinity of the antibody to a transition state analogue. No such restrictions apply to the in vitro library where catalysis can, in principle, be assayed directly. The ability to directly assay large numbers of antibodies for function may allow selection for catalysts in reactions where a mechanism is not well defined or synthesis of the transition state analog is difficult. Assaying for catalysis directly eliminates the bias of the screening procedure for reaction mechanisms pejorative to a synthetic analog and therefore simultaneous exploration of multiple reaction pathways for a given chemical transformation are possible.

The methods disclosed herein describe generation of Fab fragments which are clearly different in a number of important respects from intact (whole) antibodies. There is undoubtedly a loss of affinity in having monovalent Fab antigen binders but this can be compensated by selection of suitably tight binders. For a number of applications such as diagnostics and biosensors it may be preferable to have monovalent Fab fragments. For applications requiring Fc effector functions, the technology already exists for extending the heavy chain gene and expressing the glycosylated whole antibody in mammalian cells.

The ideas presented here address the bottle neck in the identification and evaluation of antibodies. It is now possible to construct and screen at least three orders of magnitude more clones with mono-specificity than previously possible. The potential applications of the method should span basic research and applied sciences.

## Claims

1. A method of producing a V_{H} or V_{L} catalytic-receptor-coding nucleic acid, which method comprises:
(a) synthesizing a conserved V_{H} or V_{L} receptor-coding gene library containing at least 10³ different V_{H} or V_{L} receptor-coding DNA homologs by:
(i) separating the strands of a repertoire of conserved V_{H} or V_{L} receptor-coding genes, said repertoire comprising double-stranded nucleic acids each containing a V_{H} or V_{L} receptor-coding strand annealed to a complementary strand;
(ii) treating said separated strands, under conditions suitable for polymerase chain reaction amplification, with first and second polynucleotide synthesis primers, each of said first primers having a nucleotide sequence capable of hybridizing to a sequence conserved among said V_{H} or V_{L} receptor-coding strands, and each of said second primers having a nucleotide sequence capable of hybridizing to a sequence conserved among said complementary strands, said primers being capable of priming the amplification of a plurality of different V_{H} or V_{L} receptor-coding DNA homologs from said V_{H} or V_{L} receptor-coding gene repertoire, said treating producing said conserved receptor-coding gene library; and
(b) segregating from said library a V_{H} or V_{L} receptor-coding nucleic acid coding for a catalytic receptor.

2. The method of claim 1 wherein said conserved receptor-coding nucleic acid codes for a V_{H}, said conserved receptor-coding genes are V_{H}-coding genes, and said receptor-coding DNA homologs are V_{H}-coding DNA homologs.

3. The method of claim 2 wherein said first polynucleotide synthesis primer hybridizes to an immunoglobulin J_{H} or framework region nucleotide sequence.

4. The method of claim 2 wherein said second polynucleotide synthesis primer hybridizes to a framework, leader or promoter region of a V_{H} immunoglobulin gene.

5. The method of claim 2 wherein said segregating comprises:
(a) operatively linking for expression each of a plurality of said different V_{H}-coding DNA homologs to an expression vector, thereby forming a plurality of different V_{H}-expression vectors;
(b) transforming a population of host cells compatible with said expression vector with a plurality of said different V_{H}-expression vectors to produce a transformed population of host cells whose members contain said V_{H}-expression vectors;
(c) culturing said transformed population under conditions for expressing the receptors coded for by said V_{H}-coding DNA homologs;
(d) assaying the members of said transformed population for expression of a receptor capable of binding a preselected ligand, thereby identifying transformants containing said V_{H}-coding DNA homolog; and
(e) segregating an identified transformant of step (d) from said population, thereby producing said conserved V_{H}-coding nucleic acid.

6. The method of claim 5 wherein said host cells express a V_{L} molecule, and said identified transformants express an F_{V} that binds said preselected ligand.

7. The method of claim 5 wherein all of the members of said population of host cells express the same preselected V_{L}, and said identified transformants express an F_{V} that binds said preselected ligand.

8. The method of claim 5 wherein said receptor contains a preselected epitope coded for by either of said primers or said expression vector.

9. The method of claim 5 wherein said expression vector is an episome, phage or plasmid comprised of a selectable marker gene.

10. The method of claim 1 wherein said conserved receptor-coding nucleic acid codes for a V_{L}, said conserved receptor-coding genes are V_{L}-coding genes, and said receptor-coding DNA homologs are V_{L}-coding DNA homologs.

11. The method of claim 10 wherein said first polynucleotide synthesis primer hybridizes to an immunoglobulin J_{L} or framework region nucleotide sequence.

12. The method of claim 10 wherein said second polynucleotide synthesis primer hybridizes to a framework, leader or promoter region of a V_{L} immunoglobulin gene.

13. The method of claim 1 wherein said synthesizing is performed using a plurality of different first primers.

14. The method of claim 5 wherein said expression vector molecules are linear DNA expression vector molecules.

15. The method of claim 14 wherein said linear DNA expression vector molecules are phage vector molecules.

16. The method of claim 15 wherein said phage vector molecules are Lambda Zap II V_{H} molecules, as depicted in Figure 7.

17. The method of claim 3 or 11 wherein said first polynucleotide synthesis primer encodes a predetermined restriction endonuclease recognition site.

18. The method of claim 4 or 12 wherein said second polynucleotide synthesis primer encodes a predetermined restriction endonuclease recognition site.

19. A method of producing a catalytic receptor comprising:
(a) operatively linking for expression a gene isolated according to claim 1 or 5 to a suitable expression vector to form a V_{H}-expression vector;
(b) transforming a host cell compatible with said expression vector to produce a transformant;
(c) culturing said transformant under conditions for expressing the catalytic receptor coded for by said V_{H}-coding DNA homolog, thereby producing said catalytic receptor in said culture; and
(d) recovering from said culture said catalytic receptor.

20. The method of claim 19 wherein said host cell contains a V_{L}-coding gene that expresses a V_{L} capable of modulating the catalytic activity of said produced catalytic receptor, and wherein said produced catalytic receptor is present as a part of an F_{V} comprised of said receptor and said V_{L}.

21. The method of claim 20 wherein said isolated gene and said V_{L}-coding gene are operatively linked for expression to the same expression vector.

22. The bacterial expression vector Lambda Zap II V_{H}, as depicted in Figure 7.

23. The bacterial expression vector Lambda Zap II V_{L}, as depicted in Figure 8.

## Patentansprüche

1. Verfahren zur Herstellung einer für katalytischen V_{H}- oder V_{L}-Rezeptor kodierenden Nukleinsäure, welches Verfahren umfasst:
(a) das Synthetisieren einer Bibliothek konservierter für V_{H}- oder V_{L}-Rezeptor kodierender Gene, die zumindest 10³ verschiedene für V_{H}- oder V_{L}-Rezeptor kodierende DNA-Homologe enthält, durch:
(i) Trennen der Stränge eines Repertoires konservierter für V_{H}- oder V_{L}-Rezeptor kodierender Genen, wobei das Repertoire doppelstrangige Nukleinsäuren umfasst, die jeweils einen für V_{H}- oder V_{L}-Rezeptor kodierenden Strang enthalten, der an einen komplementären Strang anneliert ist;
(ii) das Behandeln der getrennten Stränge unter Bedingungen, die für Polymerase-Kettenreaktion-Amplifizierung geeignet sind, mit ersten und zweiten Polynukleotid-Synthese-Primern, wobei jeder der ersten Primer eine Nukleotidsequenz aufweist, die in der Lage ist, an eine Sequenz zu hybridisieren, die unter den für V_{H}- oder V_{L}-Rezeptor kodierenden Strängen konserviert ist, und jeder der zweiten Primer eine Nukleotidsequenz aufweist, die in der Lage ist, an eine Sequenz zu hybridisieren, die unter den komplementären Strängen konserviert ist, wobei die Primer in der Lage sind, die Amplifizierung einer Vielzahl verschiedener für V_{H}- oder V_{L}-Rezeptor kodierender DNA-Homologe aus dem für V_{H}- oder V_{L}-Rezeptor kodierenden Genrepertoire zu primen, wobei die Behandlung die Bibliothek konservierter für den Rezeptor kodierender Gene erzeugt; und
(b) das Abtrennen einer für V_{H}- oder V_{L}-Rezeptor kodierenden Nukleinsäure, die für einen katalytischen Rezeptor kodiert, von der Bibliothek.

2. Verfahren nach Anspruch 1, worin die konservierte für Rezeptor kodierende Nukleinsäure für eine V_{H} kodiert, die konservierten für Rezeptor kodierenden Gene für V_{H} kodierende Gene sind und die für Rezeptor kodierenden DNA-Homologe für V_{H} kodierende DNA-Homologe sind.

3. Verfahren nach Anspruch 2, worin der erste Polynukleotid-Synthese-Primer an ein Immunglubulin J_{H} oder an eine Rahmenbereich-Nukleotidsequenz hybridisiert.

4. Verfahren nach Anspruch 2, worin der zweite Polynukleotid-Synthese-Primer an einen Rahmen-, Leader- oder Promotor-Bereich eines Immunglobulin-V_{H}-Gens hybridisiert.

5. Verfahren nach Anspruch 2, worin das Abtrennen Folgendes umfasst:
(a) für die Expression operatives Verbinden jedes einer Vielzahl verschiedener für V_{H} kodierender DNA-Homologe mit einem Expressionsvektor, wodurch eine Vielzahl verschiedener V_{H}-Expressionsvektoren gebildet wird;
(b) Transformieren einer Population von mit dem Expressionsvektor kompatiblen Wirtszellen mit einer Vielzahl der verschiedenen V_{H}-Expressionsvektoren, um eine transformierte Population von Wirtszellen zu erzeugen, deren Vertreter die V_{H}-Expressionsvektoren enthalten;
(c) Kultivieren der transformierten Population unter Bedingungen zur Expression der Rezeptoren, für welche die für V_{H} kodierenden DNA-Homologe kodieren;
(d) Testen der Elemente der transformierten Population auf Expression eines Rezeptors, der zur Bindung eines vorbestimmten Liganden in der Lage ist, wodurch Transformanten identifiziert werden, die das für V_{H} kodierende DNA-Homolog enthalten; und
(e) Abtrennen eines in Schritt (d) identifizierten Transformanten von der Population, wodurch die konservierte für V_{H} kodierende Nukleinsäure produziert wird.

6. Verfahren nach Anspruch 5, worin die Wirtszellen ein V_{L}-Molekül exprimieren und die identifizierten Transformanten ein F_{V} exprimieren, das den vorbestimmten Liganden bindet.

7. Verfahren nach Anspruch 5, worin alle Vertreter der Wirtszellenpopulation die gleiche vorbestimmte V_{L} binden und die identifizierten Transformanten ein Fᵥ exprimieren, das den vorbestimmten Liganden bindet.

8. Verfahren nach Anspruch 5, worin der Rezeptor ein vorbestimmtes Epitop enthält, für das entweder einer der Primer oder der Expressionsvektor kodiert.

9. Verfahren nach Anspruch 5, worin der Expressionsvektor ein Episom, ein Phage oder ein Plasmid ist, das/der aus einem selektierbaren Markergen besteht.

10. Verfahren nach Anspruch 1, worin die konservierte für Rezeptor kodierende Nukleinsäure für eine V_{L} kodiert, die konservierten für Rezeptor kodierenden Gene für V_{L} kodierende Gene sind und die für Rezeptor kodierenden DNA-Homologe für V_{L} kodierende DNA-Homologe sind.

11. Verfahren nach Anspruch 10, worin der erste Polynukleotid-Synthese-Primer an ein Immunglobulin J_{L} oder eine Rahmenbereich-Nukleotidsequenz hybridisiert.

12. Verfahren nach Anspruch 10, worin der zweite Polynukleotid-Synthese-Primer an einen Rahmen-, Leader- oder Promotor-Bereich eines Immunglobulin-V_{L}-Gens hybridisiert.

13. Verfahren nach Anspruch 1, worin das Synthetisieren unter Verwendung einer Vielzahl verschiedener erster Primer durchgeführt wird.

14. Verfahren nach Anspruch 5, worin die Expressionsvektor-Moleküle lineare DNA-Expressionsvektor-Moleküle sind.

15. Verfahren nach Anspruch 14, worin die linearen DNA-Expressionsvektor-Moleküle Phagenvektor-Moleküle sind.

16. Verfahren nach Anspruch 15, worin die Phagenvektor-Moleküle λ-Zap II-V_{H}-Moleküle sind, wie in Fig. 7 dargestellt.

17. Verfahren nach Anspruch 3 oder 11, worin der erste Polynukleotid-Synthese-Primer für eine vorbestimmte Restriktionsendonukleasen-Erkennungsstelle kodiert.

18. Verfahren nach Anspruch 4 oder 12, worin der zweite Polynukleotid-Synthese-Primer für eine vorbestimmte Restriktionsendonukleasen-Erkennungsstelle kodiert.

19. Verfahren zur Herstellung eines katalytischen Rezeptors, Folgendes umfassend:
(a) für die Expression operatives Verbinden eines gemäß Anspruch 1 oder 5 isolierten Gens mit einem geeigneten Expressionsvektor, um einen V_{H}-Expressionsvektor zu bilden;
(b) Transformieren einer Wirtszelle, die mit dem Expressionsvektor kompatibel ist, um einen Transformanten zu erzeugen;
(c) Kultivieren des Transformanten unter Bedingungen zur Expression des katalytischen Rezeptors, für den das für V_{H} kodierende DNA-Homolog kodiert, wodurch der katalytische Rezeptor in der Kultur produziert wird; und
(d) das Gewinnen des katalytischen Rezeptors aus der Kultur.

20. Verfahren nach Anspruch 19, worin die Wirtszelle ein für V_{L} kodierendes Gen enthält, das eine V_{L} exprimiert, die zur Modulation der katalytischen Aktivität des produzierten katalytischen Rezeptors in der Lage ist und worin der produzierte katalytische Rezeptor als Teil eines F_{V} vorliegt, das aus dem Rezeptor und der V_{L} besteht.

21. Verfahren nach Anspruch 20, worin das isolierte Gen und das für V_{L} kodierende Gen mit demselben Expressionvektor für die Expression operativ verbunden sind.

22. Bakterieller Expressionvektor λ-Zap II-V_{H}, wie in Fig. 7 dargestellt.

23. Bakterieller Expressionsvektor λ-Zap II-V_{L}, wie in Fig. 8 dargestellt.

## Revendications

1. Méthode de production d'un acide nucléique codant pour un récepteur catalytique de V_{H} ou V_{L}, laquelle méthode comprend:
(a) la synthèse d'une génothèque codant un récepteur de V_{H} ou V_{L} conservé contenant au moins 10³ homologues d'ADN codant un récepteur de V_{H} ou V_{L} différents par:
(i) séparation des brins d'un répertoire de gènes codant un récepteur de V_{H} ou V_{L} conservé, ledit répertoire comprenant des acides nucléiques à deux brins, chacun contenant un brin codant un récepteur de V_{H} ou V_{L} recuit à un brin complémentaire;
(ii) traitement desdits brins séparés dans des conditions appropriées pour une amplification par réaction en chaîne de polymérase, avec des premières et secondes amorces de synthèse polynucléotidiques, chacune desdites premières amorces ayant une séquence de nucléotides capable de s'hybrider à une séquence conservée parmi lesdits brins codant pour les récepteurs de V_{H} ou V_{L} et chacune desdites secondes amorces ayant une séquence de nucléotides capable de s'hybrider à une séquence conservée parmi lesdits brins complémentaires, lesdites amorces étant capables d'amorcer l'amplification d'un certain nombre d'homologues d'ADN codant le récepteur de V_{H} ou V_{L} différents dudit répertoire de gènes codant pour le récepteur de V_{H} ou V_{L}, ledit traitement produisant ladite génothèque de codage du récepteur conservé; et
(b) séparer, de ladite bibliothèque, un acide nucléique codant pour le récepteur de V_{H} ou V_{L}, codant pour un récepteur catalytique.

2. Méthode de la revendication 1 où ledit acide nucléique codant pour un récepteur conservé code pour une V_{H}, lesdits gènes codant pour le récepteur conservé sont des gènes codant V_{H}, et lesdits homologues d'ADN codant le récepteur sont des homologues d'ADN codant V_{H}.

3. Méthode de la revendication 2 où ladite première amorce de synthèse polynucléotidique s'hybride à une immunoglobuline J_{H} ou une séquence de nucléotides d'une région de charpente.

4. Méthode de la revendication 2 où ladite seconde amorce de synthèse polynucléotidique s'hybride à une région de charpente, leader ou promoteur d'un gène d'immunoglobuline V_{H}.

5. Méthode de la revendication 2 où ladite séparation comprend:
(a) l'enchaînement actif pour l'expression d'un certain nombre desdits homologues différents d'ADN codant pour V_{H} à un vecteur d'expression pour ainsi former un certain nombre de vecteurs d'expression de V_{H} différents;
(b) la transformation d'une population de cellules hôtes compatibles avec ledit vecteur d'expression avec un certain nombre desdits vecteurs d'expression de V_{H} différents pour produire une population transformée de cellules hôtes dont les membres contiennent lesdits vecteurs d'expression de V_{H};
(c) la culture de ladite population transformée dans des conditions pour exprimer les récepteurs pour lesquels codent lesdits homologues d'ADN codant pour V_{H};
(d) l'analyse des membres de ladite population transformée pour l'expression d'un récepteur capable d'une liaison à un ligand présélectionné pour identifier les transformants contenant ledit homologue d'ADN codant pour V_{H}; et
(e) la séparation d'un transformant identifié à l'étape (d), de ladite population, pour ainsi produire ledit acide nucléique codant pour V_{H} conservé.

6. Méthode de la revendication 5 où lesdites cellules hôtes expriment une molécule V_{L} et lesdits transformants identifiés expriment un Fᵥ qui lie ledit ligand présélectionné.

7. Méthode de la revendication 5 où tous les membres de ladite population des cellules hôtes expriment la même V_{L} présélectionnée et lesdits transformants identifiés expriment un Fᵥ qui lie ledit ligand présélectionné.

8. Méthode de la revendication 5 où ledit récepteur contient un épitope présélectionné pour lequel code chacune desdites amorces ou ledit vecteur d'expression.

9. Méthode de la revendication 5 où ledit vecteur d'expression est un épisome, un phage ou un plasmide composé d'un gène marqueur sélectionnable.

10. Méthode de la revendication 1 où ledit acide nucléique codant pour un récepteur conservé code pour une V_{L}, lesdits gènes codant pour le récepteur conservé sont des gènes codant pour V_{L} et lesdits homologues d'ADN codant pour le récepteur sont des homologues d'ADN codant pour V_{L}.

11. Méthode de la revendication 10 où ladite première amorce de synthèse polynucléotidique s'hybride à une immunoglobuline J_{L} ou une séquence de nucléotides d'une région de charpente.

12. Méthode de la revendication 10 où ladite seconde amorce de synthèse polynucléotidique s'hybride à une région de charpente, leader ou promoteur d'un gène d'immunoglobuline V_{L}.

13. Méthode de la revendication 1 où ladite synthèse est accomplie en utilisant un certain nombre de premières amorces différentes.

14. Méthode de la revendication 5 où lesdites molécules du vecteur d'expression sont des molécules de vecteur d'expression d'ADN linéaire.

15. Méthode de la revendication 14 où lesdites molécules du vecteur d'expression d'ADN linéaire sont des molécules de vecteur phagique.

16. Méthode de la revendication 15 où lesdites molécules de vecteur phagique sont des molécules V_{H} Lambda Zap II, comme représenté à la Figure 7.

17. Méthode de la revendication 3 ou 11 où ladite première amorce de synthèse polynucléotidique code pour un site de reconnaissance d'endonucléase de restriction prédéterminé.

18. Méthode de la revendication 4 ou 12 où ladite seconde amorce de synthèse de polynucléotidique code pour un site de reconnaissance d'endonucléase de restriction prédéterminé.

19. Méthode de production d'un récepteur catalytique comprenant :
(a) l'enchaînement actif, pour l'expression d'un gène isolé selon la revendication 1 ou 5, à un vecteur d'expression approprié pour former un vecteur d'expression de V_{H};
(b) la transformation d'une cellule hôte compatible avec ledit vecteur d'expression pour produire un transformant;
(c) la culture dudit transformant dans des conditions pour exprimer le récepteur catalytique pour lequel code ledit homologue d'ADN codant pour V_{H} pour ainsi produire ledit récepteur catalytigue dans ladite culture; et
(d) la récupération de ladite culture dudit récepteur catalytique.

20. Méthode de la revendication 19 où ladite cellule hôte contient un gène codant pour V_{L} qui exprime V_{L} capable de moduler l'activité catalytique dudit récepteur catalytique produit, et où ledit récepteur catalytique produit est présent en tant que partie d'un Fᵥ composé dudit récepteur et de ladite V_{L}.

21. Méthode de la revendication 20 où ledit gène isolé et ledit gène codant pour V_{L} sont activement enchaînés pour l'expression au même vecteur d'expression.

22. Vecteur d'expression bactérien Lambda Zap II V_{H}, tel que représenté à la Figure 7.

23. Vecteur d'expression bactérien Lambda Zap II V_{L}, tel que représenté à la Figure 8.
